# EUROPEAN PATENT SPECIFICATION

(11) **EP 2 926 731 B1**
(45) Date of publication and mention of the grant of the patent: **12.06.2024**
(21) Application number: 13858886.8
(22) Date of filing: 29.11.2013
(51) Int. Cl.: A61B 5/107, A61M 5/00, H01L 25/075, H01L 33/50, A61M 5/42, A61B 5/00

(54) **MEDICAL LIGHT SOURCE AND MEDICAL LIGHT SOURCE SYSTEM USING SAME**
MEDIZINISCHE LICHTQUELLE UND MEDIZINISCHES LICHTQUELLENSYSTEM DAMIT
SOURCE LUMINEUSE POUR APPLICATION MÉDICALE ET SYSTÈME ASSOCIÉ L'UTILISANT

(30) Priority: 30.11.2012 JP 2012262868; 28.02.2013 JP 2013039184
(43) Date of publication of application: 07.10.2015
(73) Proprietor: Seoul Semiconductor Co., Ltd., Ansan-si, Gyeonggi-do 15429 (KR)
(72) Inventor: YAMAKAWA, Masahiko, Tokyo 105-8001 (JP); ITOGA, Tatsunori, Tokyo 105-8001 (JP); SHIRAKAWA, Yasuhiro, Tokyo 105-8001 (JP)
(74) Representative: Hoffmann Eitle
(86) International application number: PCT/JP2013/082273
(87) International publication number: WO 2014/084379

(56) References cited:
- EP-A2- 1 160 883
- WO-A1-02/35994
- WO-A1-2006/049194
- GB-A- 2 347 018
- JP-A- 2000 316 866
- JP-U- 3 144 999
- US-A1- 2005 257 795

## Description

### [BACKGROUND OF THE INVENTION]

### Field of the Invention

The present invention relates to a light source for medicine and a light source system using the same.

### Background Art

Lamps are highly pervasive in our everyday lives and indispensable for leading a comfortable life. In recent years, with the advance of lightening technology, various types of products have been developed for the use in our day-to-day lives as well as in a specific field such as medicine and the like. For example, as lamps for surgery, a shadowless operating light with multiple lamps which prevents the shadow of a practitioner from forming on the affected area to be treated, an endoscope light used for the purpose of looking inside the human body, etc. have been developed. These lamps for medicine, for example, use special high color rendering lamps so that affected areas in need of surgery can be readily distinguished from healthy parts. Thus, improvement not only in the device structure and operability but also in optical application has been made.

On the other hand, even in the same medical field, special lamps are rarely used in general wards of a hospital or as indoor lighting in a medical clinic, and general household bulbs or fluorescent lamps are usually used instead. Even though these lamps are general household lamps, many of recent ones have an improved color rendering property and are given consideration so that it is possible to see, for example, people's complexions and the like in a natural way. Therefore, in performing general medical procedures, the necessity does not arise for using special light sources.

However, the medical procedures that are generally performed include, for example, injection, intravenous drip and the like. These treatments are performed by injecting a drug solution into a blood vessel, for which it is necessary to find a blood vessel part in tissues of the human body and insert an injection needle into an exact location. To that end, the color of skin has to be clearly distinguished from that of a blood vessel part in a site such as an arm.

In search of blood vessels in skin tissue, blood vessels are observed through the skin since they exist inside the skin. Blood is usually red. Blood looks red due to the existence of hemoglobin contained in blood. Skin, on the other hand, contains melanin pigment which has a dark brown hue. Therefore, as the melanin pigment content increases, the skin is more strongly colored, having a color ranging from white to brown, based on the stage. Observing blood vessels through the skin which is pigmented in this way makes the blood vessels, which should show a red color originally, look violet as a result. In a case of Japanese people, light violet blood vessels are usually observed in the skin that appears yellow.

The discernibility of blood vessels varies depending on each person. The condition of a lamp also changes the discernibility. Nonetheless, in household lamps for general purposes, the discernibility of blood vessels is not taken into account. Since household lamps are required only to represent colors of human skin truthfully, it is rather preferable that blood vessels are not noticeable. However, in limited use such as injection and intravenous drip, a lamp is desired that allows for not only the identification of skin color but also clear distinction between the skin and blood vessels. Especially, it is desired to provide a lamp which can identify readily the location of blood vessels and increase the success rate of injection or the like even when a subject is a patient with narrow blood vessels which are hard to find, such as a child.

JP 2000-30063 A (Patent Document 1) discloses a method to observe and distinguish a blood vessel pattern of an eardrum or an external auditory canal in order to identify an individual. JP 2007-87834 A (Patent Document 2) discloses a fluorescent lamp that emits near infrared rays of 700 nm.

### Prior Art Documents

### Patent Documents

Patent Document 1: JP 2000-30063 A
Patent Document 2: JP 2007-87834 A

WO 02/35994 A1 relates to a lamp for use in medicine which emits light essentially lacking the regions of 520 nm to 580 nm. GB 2 347 018 A is further prior art.

### [SUMMARY OF THE INVENTION]

### Problems to be solved by the invention

In above Patent Document 1, visible light of various wavelengths and far-red light are disclosed as effective light sources for identifying a blood vessel pattern. Especially, the far-red light having a wavelength of 930 nm is described as the most effective light source. Because the far-red light of 930 nm is absorbed only a little by skin pigments, such as melanin, bilirubin and beta-carotene but absorbed by oxygenated hemoglobin and reduced hemoglobin in blood, once the far-red light is irradiated on the human body and penetrates subcutaneous tissue, it reflects the blood vessel pattern when returning to the skin tissue after reflection and diffusion. Thus, the light source according to Patent Document 1 is useful in the observation of a blood pattern, but far-red light is not visible and cannot be observed with naked eyes. As a result, the light source has a structure that needs a special evaluation device, e.g., using a CCD in a light receiving unit. In Patent Document 1, it is inevitable to employ a special evaluation device because accurate data are required. However, for blood vessel observation during an injection, an intravenous drip or the like, the accuracy capable of being used as identification of an individual is not required, but instead, the light source has to be available daily in an easy way without using a special evaluation device.

The fluorescent lamp according to Patent Document 2 is one of lamps which are used daily in a general household, but differs from the present invention in its intended use. In Patent Document 2, the emission color of the device is determined in consideration of the light absorption by hemoglobin. Therefore, the fluorescent lamp of Patent Document 2 is a lamp which is characterized in that light is not absorbed by hemoglobin and which is aimed at beauty, and shares little common purpose with the present invention.

The present invention has an object to provide a light source for medicine which can improve the visibility of blood vessels in the skin. Specifically, the present invention is accomplished by focusing on making the difference bigger between the reflectivity by the light from melanin, which is a component of skin color, and the reflectivity by the light from hemoglobin, which is a component of blood color. In other words, by irradiating light that can decrease the intensity of reflected light from melanin and enhance the intensity of reflected light from hemoglobin, the present invention strives to make the difference of the both stand out.

### Means for solving the problems

### The invention is defined by independent claim 1.

The light source for medicine according to the present invention is characterized in that, in an emission spectrum of a white light source for medicine, a P2 to P3 ratio (P3/P2) is 0.4 or less, wherein P3 is the maximum intensity of the emission spectrum within a wavelength region from 520 nm to 580 nm, and P2 is the maximum intensity of the emission spectrum within a wavelength region from 600 nm to 780 nm.

According to the present invention, in an emission spectrum of the light source, the emission intensity of a yellow region (520 to 580 nm) is decreased and the emission intensity of a red region (600 to 780 nm) is increased, thereby suppressing the emission intensity of the former in respect to the latter to be 0.4 or less. Thus, a white light source for medicine can be provided which has an improved visibility of blood vessels in the skin. This enables the location of blood vessels in the skin to be exactly identified and can enhance the success rate of injection or the like into the blood vessel.

The light source for medicine according to the present invention is characterized in that the emission spectrum thereof has emission peaks within a wavelength region from 370 nm to 470 nm and 600 nm to 780 nm respectively, and that a P1 to P2 ratio (P1/P2) is 0.5 or more and 1.2 or less, wherein P1 is the maximum intensity of the emission spectrum within a wavelength region from 370 nm to 470 nm and P2 is the maximum intensity of the emission spectrum within a wavelength region from 600 nm to 780 nm.

According to the present invention, the emission spectrum of the light source has emission peaks within a wavelength region of 370 nm to 470 nm and 600 nm to 780 nm respectively, and controls the ratio of maximum intensity of the emission spectrum within a wavelength region from 370 nm to 470 nm to the emission spectrum within a wavelength region from 600 nm to 780 nm, thereby making it possible to increase the visibility of blood vessels in the skin. Furthermore, the emission spectrum of the light source has certain emission intensity within a wavelength region from 370 nm to 470 nm, which can make the emission color of the light source less red and decrease the glare. As a result, the location of blood vessels in the skin can be identified accurately and the success rate of injection or the like into a blood vessel can be increased.

As another embodiment, the present invention further provides a light source system for medicine comprising the above light source for medicine.

### [BRIEF DESCRIPTION OF THE DRAWINGS]

FIG. 1 is a cross-sectional diagram illustrating an embodiment of the light source for medicine of the present invention.
FIG. 2 is a schematic diagram illustrating another embodiment of the light source for medicine of the present invention.
FIG. 3 is a diagram illustrating an emission spectrum of the light source for medicine of Example A1.
FIG. 4 is a diagram illustrating an emission spectrum of the light source for medicine of Example A2.
FIG. 5 is a diagram illustrating an emission spectrum of the light source for medicine of Example A3.
FIG. 6 is a diagram illustrating an emission spectrum of the light source for medicine of Example A4.
FIG. 7 is a diagram illustrating an emission spectrum of the light source for medicine of Example A5.
FIG. 8 is a diagram illustrating an emission spectrum of the light source for medicine of Example A6.
FIG. 9 is a diagram illustrating an emission spectrum of the light source for medicine of Example A7.
FIG. 10 is a diagram illustrating an emission spectrum of the light source for medicine of Example A8.
FIG. 11 is a diagram illustrating an emission spectrum of the light source for medicine of Example A9.
FIG. 12 is a diagram illustrating an emission spectrum of the light source for medicine of Example A10.
FIG. 13 is a diagram illustrating an emission spectrum of the light source for medicine of Example A11.
FIG. 14 is a diagram illustrating an emission spectrum of the light source for medicine of Example A12.
FIG. 15 is a diagram illustrating an emission spectrum of the light source for medicine of Example A13.
FIG. 16 is a diagram illustrating an emission spectrum of the light source for medicine of Example A14.
FIG. 17 is a diagram illustrating an emission spectrum of the light source for medicine of Example A15.
FIG. 18 is a diagram illustrating an emission spectrum of the light source for medicine of Example A16.
FIG. 19 is a diagram illustrating an emission spectrum of the light source for medicine of Example A17.
FIG. 20 is a diagram illustrating an emission spectrum of the light source for medicine of Example A18.
FIG. 21 is a diagram illustrating an emission spectrum of the light source for medicine of Comparative Example A1.
FIG. 22 is a diagram illustrating an emission spectrum of the light source for medicine of Comparative Example A1.
FIG. 23 is a diagram illustrating an emission spectrum of the light source for medicine of Example B1 not falling under claim 1.
FIG. 24 is a diagram illustrating an emission spectrum of the light source for medicine of Example B2.
FIG. 25 is a diagram illustrating an emission spectrum of the light source for medicine of Example B3.
FIG. 26 is a diagram illustrating an emission spectrum of the light source for medicine of Example B4.
FIG. 27 is a diagram illustrating an emission spectrum of the light source for medicine of Example B5.
FIG. 28 is a diagram illustrating an emission spectrum of the light source for medicine of Example B6 not falling under claim 1.
FIG. 29 is a diagram illustrating an emission spectrum of the light source for medicine of Example B7 not falling under claim 1.
FIG. 30 is a diagram illustrating an emission spectrum of the light source for medicine of Example B8.
FIG. 31 is a diagram illustrating an emission spectrum of the light source for medicine of Example B9.
FIG. 32 is a diagram illustrating an emission spectrum of the light source for medicine of Example B10.
FIG. 33 is a diagram illustrating an emission spectrum of the light source for medicine of Example B11.
FIG. 34 is a diagram illustrating an emission spectrum of the light source for medicine of Example B12.
FIG. 35 is a diagram illustrating an emission spectrum of the light source for medicine of Example B13.
FIG. 36 is a diagram illustrating an emission spectrum of the light source for medicine of Example B14.
FIG. 37 is a diagram illustrating an emission spectrum of the light source for medicine of Example B15.
FIG. 38 is a diagram illustrating an emission spectrum of the light source for medicine of Example B16.
FIG. 39 is a diagram illustrating an emission spectrum of the light source for medicine of Example B17.
FIG. 40 is a diagram illustrating an emission spectrum of the light source for medicine of Example B18.
FIG. 41 is a diagram illustrating an emission spectrum of the light source for medicine of Example B19.
FIG. 42 is a diagram illustrating an emission spectrum of the light source for medicine of Example B20.
FIG. 43 is a diagram illustrating an emission spectrum of the light source for medicine of Example B21.
FIG. 44 is a diagram illustrating an emission spectrum of the light source for medicine of Comparative Example B2.

### [DETAILED DESCRIPTION OF THE INVENTION]

As an example, the light source for medicine according to the disclosure may be characterized in that, in the emission spectrum of the light source, a P3 to P2 ratio (P3/P2) is 0.19 or less, wherein P3 is the maximum emission intensity of a yellow region (520 to 580 nm) and P2 is the maximum emission intensity of a red region (600 to 780 nm). Upon irradiation on a human arm, leg or the like by such a light source, it is possible to distinguish blood vessels in the skin easily by suppressing the emission intensity of a yellow region to be a certain value or less in respect to that of a red region. This is because melanin pigment in the skin is pigmented in brown and the light reflection intensity of a yellow wavelength region is high while blood has strong light reflection intensity a red wavelength region because hemoglobin contained therein has a red color. In other words, because melanin pigment or hemoglobin itself does not emit light in brown and in red respectively, among light which is irradiated towards the melanin pigment or hemoglobin, the ratio of yellow light to be reflected by melanin pigment decreases when the ratio of a yellow-emitting component is low while the ratio of red light to be reflected by hemoglobin increases when the ratio of a red-emitting component is high.

The discernibility of blood vessels in the skin varies depending on the relative balance between the reflection light intensity from the skin and that from blood vessels. Some people have thick blood vessels and others have thin blood vessels. As for skin color as well, according to the content of melanin pigment, some have a skin color close to white while others have a skin color close to brown. Accordingly, the discernibility of blood vessels varies depending on each person. The above-mentioned standard (P3/P2) ratio is not universal to everybody. The standard value of 0.19 or less as described above shows a boundary value capable of the exact assessment aimed at average patients. A trained nurse could distinguish blood vessels of at least 99 patients out of 100 patients. However, in order for regular nurses to distinguish blood vessels without any error, it is desirable to be able to identify the blood vessels more distinctly. Therefore, the above (P3/P2) ratio is preferred to be 0.15 or less.

Even though the light source for medicine of the present invention is used, it is still difficult to identify the blood vessels of 100% of all the patients. For infrequently-existing, unusual patients, not only the use of the light source of the present invention but also the addition of further effort such as expanding blood vessels by adding pressure on an arm, are needed.

The effect of the present invention is demonstrated by identifying the shape of an emission spectrum as described above and is not affected by the type of light source, the structure of an emission device or the like. Accordingly, in order to embody the present invention, any means can be used. An example that is considered to be the most effective method, but not limited thereto, will be given below to describe the content of the present invention in more detail.

As a method to obtain a light source having the characteristics according to the prevent invention, a method to use a phosphor material(s) is the easiest and desirable as well in terms of the properties thereof. For phosphor materials, various types of products at a practical level have been developed, and it is possible to select arbitrarily a combination among numerous phosphor materials when obtaining an arbitrary shape of an emission spectrum. Especially, among phosphor types, some change the emission colors continuously when, even though the elemental compositions are the same, the ratios of elements constituting the parent bodies are altered or when the concentration of an activator is altered. Therefore, it is not too much to say that combinations of practical phosphors can represent almost all the wavelengths in a region of visible light. Moreover, in the use of phosphors, various mixed lights can be obtained readily by mixing powder to fine tune the emission colors. As a result, the emission color as designed can be achieved, which is desirable in terms of the characteristics as well.

The emission of a phosphor needs an excitation light source, and in a case for lighting, ultraviolet rays and blue light are commonly used. Examples of the excitation light source include emission devices such as a fluorescent lamp using ultraviolet rays and an LED lamp using ultraviolet rays or blue light. For the light source for medicine of the present invention, either may be used. Both of them do not need special equipment or a large-scale device and can be lighted by using a general lighting apparatus commercially available. Moreover, as for each emission spectrum as well, an arbitrary shape thereof can be obviously obtained. Thus, although both of a fluorescent light and an LED lamp are equivalent in achieving the effect of the present invention, the LED lamp has superior characteristics comprehensively because it has a longer lifespan and energy-saving effect, and further has an advantage such as easy miniaturization and the like. The description thereinafter will focus on the LED lamp.

For the LED used in the light source for medicine according to the present invention, an LED that emits ultraviolet light, violet light or blue light can be used. It is preferable that the spectrum of the light emitted from the LED has a peak wavelength of 370 nm or more and 480 nm or less. Examples of LEDs that exhibit such a range of emission spectrum include InGaN-based, GaN-based and AlGaN-based light emitting diode chips.

For a phosphor(s) to be combined with an LED, it is preferred to use a phosphor(s) that emit(s) brightly three primary colors, red, blue or green respectively when excited by the light source of 370 nm to 480 nm. An arbitrary number of types of phosphors can be used as long as it is one or more. Accordingly, a phosphor(s) that emit(s) light in intermediate colors such as blue green, yellow or dark red color in addition to the three primary colors of red, blue or green can be used. In terms of the natural representation of objects, the more types of phosphors are used, the more desirable it is. However, the present invention is characterized by suppressing the intensity of yellow emission and it is necessary to be aware of the upper limit of the mixing amount when a yellow-emitting phosphor is mixed. Moreover, in order to obtain a bright light source, each component phosphor needs to emit brightly individually, but the more types of phosphors are mixed, the harder it becomes to select bright phosphors. Generally, therefore, it is preferred to mix about 2 to 5 types.

As the types of phosphors available in the present invention, in terms of brightness and emission colors thereof, the following phosphors, but not limited to, are preferably used. As blue phosphors, europium-activated alkaline earth halophosphate phosphor, europium-activated alkaline earth aluminate phosphor, europium-activated strontium pyro phosphor, europium-activated strontium magnesium pyro phosphor, europium-activated alkaline earth silicate phosphor or the like is preferably used; as green phosphors, europium-activated alkaline earth ortho silicate magnesium phosphor, europium-activated sialon phosphor, europium- and manganese-activated alkaline earth aluminate phosphor, cerium- and terbium-activated phosphate lanthanum phosphor, cerium- and terbium-activated silicate yttrium phosphor, copper-activated zinc sulfide phosphor or the like is preferably used; and as red phosphors, europium-activated sialon phosphor, europium-activated CASN phosphor, europium-activated lanthanum oxysulfide lanthanum phosphor, europium-activated yttrium oxide phosphor or the like is preferably used.

Furthermore, for phosphors which emit in intermediate colors, as blue green phosphors, europium-activated alkaline earth halophosphate phosphor, europium-activated alkaline earth aluminate phosphor, europium-activated alkaline earth ortho silicate phosphor, tin-activated strontium pyro phosphor or the like is preferably used; as yellow-emitting phosphors, europium-activated alkaline earth ortho silicate magnesium phosphor, europium-activated sialon phosphor, cerium-activated aluminate yttrium phosphor or the like is preferably used; and as dark red phosphors, manganese-activated fluoro germanate phosphor or the like is preferably used. About 1 to 5 types of phosphors among the phosphors of three primary colors and intermediate colors can be mixed at an arbitrary ratio, thereby to obtain white emission having high luminance, a high color rendering property, and various color temperatures.

Although the present invention relates to a light source for medicine, the light source color is not necessarily white. For example, it may be a mixed light including two colors of a yellow component and a red component, wherein the light source is red or orange. In the case of red light or orange, there is no problem in only identifying the skin and blood vessels. However, the light source is most preferred to be white light due to occurrence of a problem that the color of an object cannot be seen in a natural way.

On the other hand, the present invention is not only used to illuminate an entire room. There are cases where the present invention is used as a lamp to illuminate the area around a hand. For example, while a regular fluorescent lamp or the like is used for the entire room, the light source of the present invention is located on a table or a stool for injection and used as a handy lamp. In this case, general objects are represented in natural colors by the room lamp and only an arm or the like which is a site of interest for injection is irradiated by the light source of the present invention. Therefore, there is evidently no problem even if red light or orange light is used.

The type(s) and the amount(s) of a phosphor(s) to be mixed are determined when the light source color of interest or the color temperatures in the case of a white light source is determined.

A phosphor(s) is (are) mixed with a transparent resin such as a silicone resin and the like to form a phosphor slurry, and the phosphor slurry is coated on a circumference of an LED chip to form a phosphor film. The structure or shape of the phosphor film is not specifically limited, and any structure or shape can be used. However, when the LED chip emits ultraviolet light, it is preferred that the entire surface of the chip is covered with the phosphor film and the film thickness of the phosphor film is at least 0.07 mm or more. If a part of the LED chip is uncovered with the phosphor film, the direct light from the LED leaks outside the light source. Likewise, when the film thickness of the phosphor film is less than 0.07 mm, a part of the LED light which is directed towards the phosphor film is absorbed by the phosphor film while the rest part penetrates the phosphor film and results in leaking outside the light source. In the case of an ultraviolet-emitting LED, the leakage of ultraviolet rays outside the light source causes a concern about bad influence on the human body. Furthermore, the ratio of the LED light which is absorbed by a phosphor(s) and converted to visible light is reduced and the luminance of the light source decreases.

It is known that, in a relationship with the brightness of a phosphor film, the optimal film thickness of a phosphor film gets thicker as the particle size of the phosphor powder increases and gets thinner as the particle size of the phosphor powder decreases. When the film thickness of a phosphor film is at least 0.07 mm or more, the case is assumed in which the particle size of the phosphor(s) used in the present invention is the smallest, more specifically, the particle size of the phosphor(s) is 2 to 3 µm. As the average particle size of the phosphor(s) to be used increases, the film thickness of the phosphor(s) also gets thicker corresponding to the degrees of increase. For example, when a phosphor(s) with the average particle size of 10 µm is (are) used, the film thickness of the phosphor(s) is preferred to be 0.1 mm or more.

When the LED to be used is a blue LED, it is not required to prevent the LED light from leaking outside the light source and the minimum film thickness of a phosphor film does not have to be limited. When a blue LED is used, direct light from the LED is usually designed so that a part thereof is used to excite a phosphor(s) while another part thereof is used as a blue component of the white light source, and it is rather important that the light is taken outside the light source. Therefore, in the case of a blue LED, it is preferred that the film thickness of a phosphor film is a certain value or less. However, even when a blue LED is used, if the approximately all amount of the LED light is designed to be absorbed by the phosphor film, i.e., in an entire surface conversion way, it is preferred that the LED light does not leak outside the light source and that, as with an ultraviolet LED, the film thickness is a certain value or more

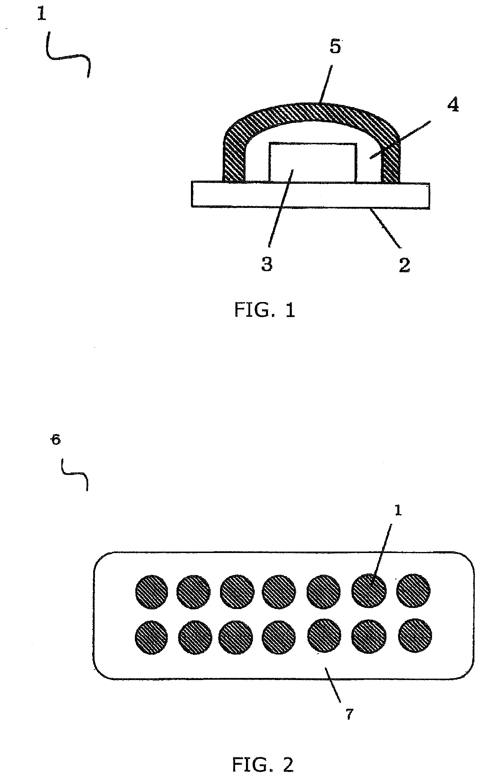

A phosphor film may be directly coated on the surface of an LED chip, but it is preferable that a transparent resin layer is formed between an LED chip and a phosphor film. Fig. 1 illustrates one embodiment of the light source for medicine according to the present invention. In the figure, 1 is a light source for medicine, 2 is a substrate, 3 is an LED, 4 is a transparent resin layer and 5 is a phosphor layer.

Examples of the substrate 2 include those having insulation properties, for example, ceramic substrates such as an alumina substrate and insulating resin substrates. One or plural LED(s) 3 may be used. The transparent resin layer 4 is provided as required. The phosphor layer 5 is a film having a phosphor(s). Examples of the phosphor layer includes a mixture of one, two or more types of phosphors and a resin. The LED3 is conducted by wiring which is not illustrated.

As illustrated in Fig.1, a transparent resin layer 4 may be formed between a phosphor film 5 and an LED chip 3. The end to form the transparent resin layer 4 between the LED 3 and the phosphor layer 5 is to increase the emission intensity of the light source. Although most of the excitation light emitted from the LED is absorbed by the phosphor layer, a part thereof penetrates the phosphor layer and leaks outside the light source, and another part thereof is reflected on the phosphor layer and results in returning inside the light source. At that time, if the phosphor layer has a thickness of a certain value or more, then the ratio of the excitation light returning inside the light source increases. When the light which returns inside the light source reaches the LED, the light is confined therein to cause energy loss. This is because LED is a high refractive index material and the light no longer goes outside the chip (LED) once the light returns to the inside. In this case, if space comprising a transparent resin layer exists between the LED and the phosphor layer, the probability that light reflected on the phosphor layer is directed towards the LED decreases, and the light is reflected on a substrate or the like and directed towards the phosphor layer again, resulting in the improvement in the brightness of the phosphor layer. Moreover, by providing the transparent resin layer 4, the effect is expected to increase the emission intensity and to prevent heat deterioration.

For a transparent resin layer, it is preferred to use a material with good thermal resistance such as a silicone resin and the like. This is for the prevention of coloration or the like due to heat deterioration because an LED chip generates heart during operation and the temperature increases to about 200 °C when it is lighted continuously. In this way, a white light source having an advantage of long-term reliability can be provided. In the figure, an emission device is illustrated in which a single LED chip is covered with a transparent resin film and a phosphor film; however, it may be structured to cover a plurality of LED chips with a common transparent resin layer and a common phosphor film.

The effect described above by a transparent resin layer can be achieved when the film thickness of the resin layer is at least 0.1 mm or more. On the other hand, the film thickness of the resin layer which is too thick and is more than 1.0 mm is not preferable because, when penetrating inside the transparent resin layer, light is absorbed by the resin layer itself and energy loss is caused. Therefore, the thickness of the transparent resin layer 4 is preferred to be 0.1 to 1.0 mm. Examples of the transparent resin layer include a silicone resin.

In Fig. 1, an emission device is illustrated in which a single or plural of LED chip(s) is (are) covered with a single resin layer and a phosphor layer; however, an emission system combining a plurality of emission devices like this may be used. Fig. 2 illustrates a system in which a plurality of light sources for medicine according the present embodiment are arranged. In the figure, 1 is a light source for medicine according to the present embodiment, 6 is a light source system for medicine and 7 is a mounting substrate, and an emission system in which a plurality of LED chips are mounted on a single alumina substrate and a plurality of phosphor layers are formed each of which corresponds to each LED chip. When a plurality of light sources for medicine are used, the substrate 2 and the mounting substrate 7 may be the same or provided separately. In the emission system of Fig. 2, a number of light sources can be connected in a single circuit to obtain an emission device which emits bright light. An emission system capable of adjusting light and color simultaneously can be also obtained by using a system in which each light source is connected to a power supply in an individual circuit and the emission color of each phosphor layer is individually changed. When using a combination of a blue LED and a red LED without using a phosphor layer, it is preferable to design in a way that the current value applied to each can be changed.

Other than the embodiment illustrated in Fig. 2, a system is also available that includes a light source for medicine as a first light source for medicine and a second light source which has an emission spectrum different than the first light source for medicine. A white light source is preferred as the second light source. It is also preferred that the lighting of the first light source for medicine and the second white light source can be switched. The first light source for medicine is effective as a lamp for identifying blood vessels as described above. On the other hand, by using as the second white light source a lamp which can be used as a general lamp such as an indoor lamp, it is possible to light the first light source for medicine when the operation of blood vessel identification is performed (for example, injection and intravenous drip) and to light the second white light source during the normal operation. Consequently, in a single light source system for medicine, the normal operation and the operation of blood vessel identification can be performed.

As another example, the light source for medicine according to the disclosure may be characterized in that the emission spectrum of the light source for medicine has emission peaks within a wavelength region from 370 nm to 470 nm and 600 nm to 780 nm, respectively, and in that a P1 to P2 ratio (P1/P2) is 0.3 or more, wherein P1 is the maximum intensity of the emission spectrum within a wavelength region from 370 nm to 470 nm and P2 is the maximum intensity of the emission spectrum within a wavelength from 600 nm to 780 nm.

Firstly, the emission spectrum of the light source for medicine according to the present embodiment has emission peaks within a wavelength region from 370 nm to 470 nm and 600 nm to 780 nm, respectively. Blood contains hemoglobin. Hemoglobin is a component contained in red blood cells and has a red color. Therefore, it has high reflection intensity in a red wavelength region. The light source for medicine of the present embodiment has an emission peak within a wavelength region from 600 nm to 780 nm, i.e., within a red wavelength region to reflect the red color of hemoglobin As a result, understanding of the location of blood vessels, i.e., the visibility of blood vessels is enhanced.

The emission spectrum of the light source for medicine according to the present embodiment also has an emission peak within a wavelength region from 370 nm to 470nm. The light of 370 to 470 nm is light between violet and blue. The light source for medicine of the present embodiment is characterized in that a P1 to P2 ratio (P1/P2) is 0.3 or more wherein P1 is the maximum intensity of the emission spectrum within a wavelength region from 370 nm to 470nm and P2 is the maximum intensity of the emission spectrum within a wavelength from 600 nm to 780 nm. In other words, in respect to the emission peak P2 of a red region (600 to 780 nm), a certain amount of emission between violet and blue is produced. When the ratio (P1/P2) is 0.3 or more, red color, which is the emission color of the light source for medicine, is reduced and gets close to white. As a result, in addition to the improvement of the visibility of blood vessels, a light source with its glare reduced can be provided.

The upper limit of the ratio (P1/P2) is not particularly limited, but it is according to the invention 1.2 or less. When the ratio (P1/P2) is greater than 1.5, the amount of light within a red region may reduce relatively and the visibility of blood vessels may decrease. In order to ensure both of the improvement of the visibility of blood vessels and the reduction of the glare, the ratio (P1/P2) is according to the invention 0.5 to 1.2.

A S2 to S1 ratio (S2/S1) is preferably 0.3 or less (including zero), wherein S1 is the area of the emission spectrum within a wavelength from 370 nm to 780 nm and S2 is the area of the emission spectrum within a wavelength region from 471 to 599 nm. The ratio (S2/S1) of 0.3 or less indicates that, in respect to the entire emission light spectrum (370 to 780 nm), the ratio of the emission spectrum from yellow to green (471 to 599nm) is controlled to a certain value or less. The ratio of the emission spectrum from yellow to green (471 to 599nm) can be controlled to be a certain value or less to further enhance the characteristics of the emission peaks from 370 nm to 470 nm and 600 nm to 780 nm. The ratio (S2/S1) is preferably 0.20 or less (including zero). Although the lower limit of the ratio (S2/S1) is not particularly limited, it is preferably 0.05 or more. The emission of the light source for medicine can be made close to white by including light within a wavelength region from 471 to 599 nm in the emission spectrum, and the glare of the emission color can be further reduced.

A P3 to P2 ratio (P3/P2) is according to the invention 0.4 or less (including zero), wherein P3 is the maximum intensity of the emission spectrum within a wavelength region from 520 nm to 580 nm. The wavelength from 520 to 580 nm is emission from yellow to green. This wavelength of 520 to 580 nm is an area in which the reflectivity in the blood is low. Therefore, by not having a large emission peak at 520 to 580 nm, i.e., having the ratio (P3/P2) of 0.4 or less, characteristics of the emission peaks from 370 nm to 470 nm and 600 nm to 780 nm can be further enhanced. Besides, melanin pigment contained in the skin has a reflectivity of light at 520 to 580 nm which is 2 to 3 times higher than the reflectivity in blood. Accordingly, the visibility of blood vessels decreases when the wavelength from 520 to 580 nm is too high. Furthermore, the melanin pigment is a dark brown pigment. Therefore, when applied to a light source for medicine for visually identifying blood cells in the skin which is rich in melanin pigment, the ratio (P3/P2) is according to the invention 0.4 or less. More preferably, the ratio (P3/P2) is 0.25 or less. In addition, when the ratio (S2/S1) of 0.3 or less and the ratio (P3/P2) of 0.4 or less are combined, the effect of the improvement in visibility of blood vessels and the reduction of the glare of emission color can be further achieved.

The light source for medicine of the present embodiment preferably comprises an LED chip having an emission peak within a wavelength region from 370 nm to 470 nm and a phosphor layer which is excited by a primary light emitted from the LED chip and emits a secondary light within a visible light region. The ratio (P1/P2) can be adjusted to be 0.3 or more by using a blue LED and a red LED. As for the control of the ratio (P1/P2), the peak ratio can be adjusted by changing each current value applied to the blue LED and the red LED. On the other hand, just with the combination of a blue LED and a red LED, it is difficult to control the above ratio (S2/S1) within the range of 0.05 to 0.3. Therefore, the light source for medicine of the present invention comprises a phosphor layer in order to have the emission color of the light source for medicine which is close to white and has reduced glare.

Together with the phosphor layer, a LED chip is used which has an emission peak within a wavelength region from 370 nm to 470 nm; it is more preferably to use an LED chip which has an emission peak within a wavelength region from 370 nm to 420 nm.

For a phosphor layer, a peak wavelength of an LED to be used can be determined according to the emission spectrum of interest (P1/P1, further, S2/S1 and P3/P2) and the phosphor to be used may be selected as appropriate from the above mentioned phosphors depending on the peak wavelength of the LED. For example, the wavelength region from 421 to 470 nm causes blue emission. Therefore, P1 is an emission peak of a blue LED. When a blue LED is used, the ratio (P1/P2) can be controlled by adjusting the amount used of a red phosphor emitting in red in this blue light. Such a red phosphor can be selected as appropriate from phosphors mentioned above.

The wavelength region from 370 to 420 nm emits light in violet (including ultraviolet). When a violet-emitting LED is used, P1 can be an emission peak of a blue-emitting phosphor by using a blue-emitting phosphor. When P1 is a peak of the blue-emitting phosphor, the ratio (P1/P2) can be controlled by adjusting the amount used of a blue-emitting phosphor and a red-emitting phosphor which emits light in red due the violet-emitting LED. Such a red phosphor can be selected as appropriate from phosphors mentioned above.

When a phosphor layer is used, examples of the method to control the ratio (S2/S1) and the ratio (P3/P2) include a method to use a blue phosphor or a red phosphor which has a broad emission peak with a large half-value width. A method to use a yellow phosphor or a green phosphor in addition to a blue phosphor or red phosphor is also included. As such phosphors, similar phosphors to those used in the light source for medicine according to the first embodiment can be used. Particularly, the ratio (P1/P2) can be controlled by adjusting the mixing ratio of a red phosphor to a blue phosphor. In addition, by using phosphors which have emission peaks at 471 to 599 nm, more specifically within 520 to 580 nm such as a green phosphor, blue green phosphor, yellow phosphor and the like, the ratio (S2/S1) and the ratio (P3/P2) can be controlled. Such phosphors can be selected as appropriate from various phosphors mentioned above to be used.

In the light source for medicine of the present embodiment, the light source structure is not particularly limited as long as it has the emission spectrum with the ratio (P1/P2), the ratio (S2/S1) and the ratio (P3/P2) thereof within the above-mentioned ranges; however, preferable examples thereof include structures illustrated in Figs. 1 and 2, as mentioned above.

As described above, a light source for medicine and a light source system for medicine comprising a substrate and an LED chip, and further a mixed phosphor film and a transparent resin layer can implement a lamp that has an adjusted emission spectrum shape of the light source, allows for easy identification of blood vessels in the skin, and emits bright light. As a result, in performing an injection or an intravenous drip, the light source for medicine and the light source system for medicine aids in improving the success rate of placement of an injection needle into a blood vessel. Furthermore, the light source for medicine according to the present invention is characterized in that it can be lighted by a general lightening apparatus commercially available without the need of any special equipment or the like and has an advantage that it can be used easily anytime and anywhere in the medical field.

### [EXAMPLES]

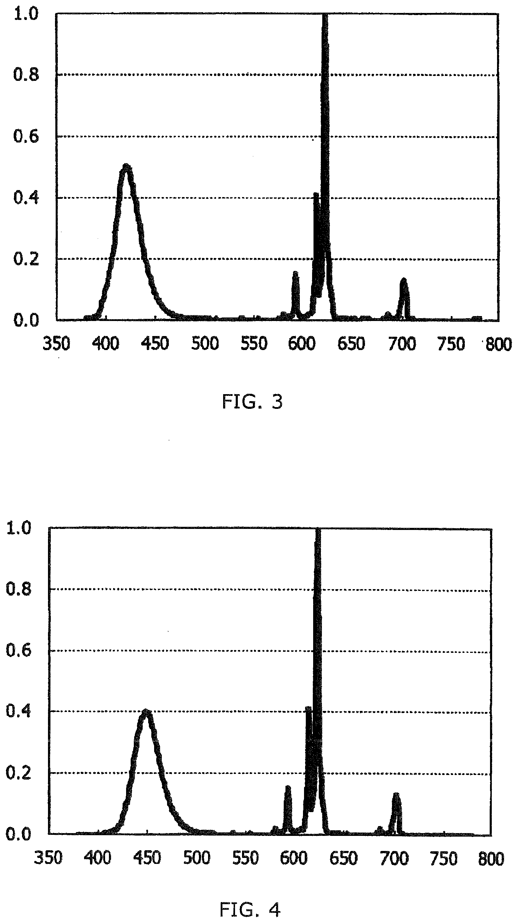

The light source for medicine and light source system for medicine of the present invention will now be described in detail by way of examples thereof. In the emission spectra shown in Figs. 3 to 44, the ordinate indicates the emission intensity (a.u.) and the abscissa indicates the wavelength (nm).

As the LED chips, those having emission peaks at 370 to 410 nm were provided. As the phosphors which receive excitation light from the LED chip and emit a visible light, six types of phosphors were provided. More specifically, as blue phosphors, europium-activated alkaline earth metal halophosphate and europium-activated strontium pyro phosphor were provided; as a blue green phosphor, europium-activated alkaline earth metal aluminate phosphor was provided; as green phosphors, europium- and manganese-activated alkaline earth metal aluminate phosphor were provided; as a yellow phosphor, europium-activated alkaline earth metal ortho silicate magnesium phosphor was provided; and as red phosphors, europium-activated lanthanum oxysulfide phosphor, manganese-activated magnesium fluoro germanate phosphor, and europium-activated sialon phosphor were provided. The specific chemical compositions of the respective phosphors were as follows:
blue phosphor 1: (Sr_{0.9}Ba_{0.03}Ca_{0.01}Eu_{0.06})₅(PO₄)₃·Cl
blue phosphor 2: (Sr_{0.9}Eu_{0.1})₂P₂O₇
blue green phosphor 1: (Sr_{0.9}Eu_{0.1})₄Al1₄O₂₅
green phosphor 1: (Ba_{0.9}Eu_{0.1})(Mg_{0.4}Mn_{0.6})Al₁₀O₁₇
yellow phosphor 1: (Sr_{0.41}Ba_{0.5}Mg_{0.03}EU_{0.05}Mn_{0.01})₂SiO₄
red phosphor 1: (La_{0.9}Eu_{0.1})₂O₂S
red phosphor 2: 3.5MgO·0.5MgF₂·(Ge_{0.9}Mn_{0.1})O₂
red phosphor 3: (Sr_{0.92}Eu_{0.08})Si₆Al₂O₂N₁₂

In the following Examples, the above-described 8 types of phosphors were mixed at various ratios to obtain white light sources having various emission spectra. In each Example, the respective phosphors are designated by combination of the color of the emitting light and the number, such as "blue 1" and "red 2".

### (Example A1)

As an LED chip, one having an emission peak wavelength at 370 nm was provided and arranged on an alumina substrate. Then a mixed phosphor composed of blue 2 and red 1 was provided. The average particle size of the respective phosphors was about 18 µm. The mixing ratio of the phosphors was, blue 2: red 1 = 30:70 by weight (by mass). The mixed phosphor was mixed with a transparent resin (silicone resin) and the resulting mixture was coated on the LED chip provided with a transparent resin layer (thickness 0.01 mm) to obtain a white light source shown in Fig. 3. The thickness of the phosphor layer was 0.18 mm. Then the emission spectrum of the white light source was measured by using a total luminous flux measuring apparatus equipped with an integrating sphere according to JIS-C-8152. In the emission spectrum of the mixed phosphor, the ratio (P3/P2) was 2%, wherein P3 was the maximum emission intensity within the yellow region (520 to 580 nm) and P2 was the maximum emission intensity within the red region (600 to 780 nm).

### (Example A2)

As an LED chip, one having an emission peak wavelength at 390 nm was provided and arranged on an alumina substrate. Then a mixed phosphor composed of blue 1 and red 1 was provided. The average particle size of the respective phosphors was about 13 µm. The mixing ratio of the phosphors was, blue 1: red 1 = 40:60 by weight (by mass). The mixed phosphor was mixed with a transparent resin (silicone resin) and the resulting mixture was coated on the LED chip provided with a transparent resin layer (thickness 0.05 mm) to obtain a white light source shown in Fig. 4. The thickness of the phosphor layer was 0.15 mm. Then the emission spectrum of the white light source was measured according to Example A1. In the emission spectrum of the white light source, the ratio (P3/P2) was 2%.

### (Example A3)

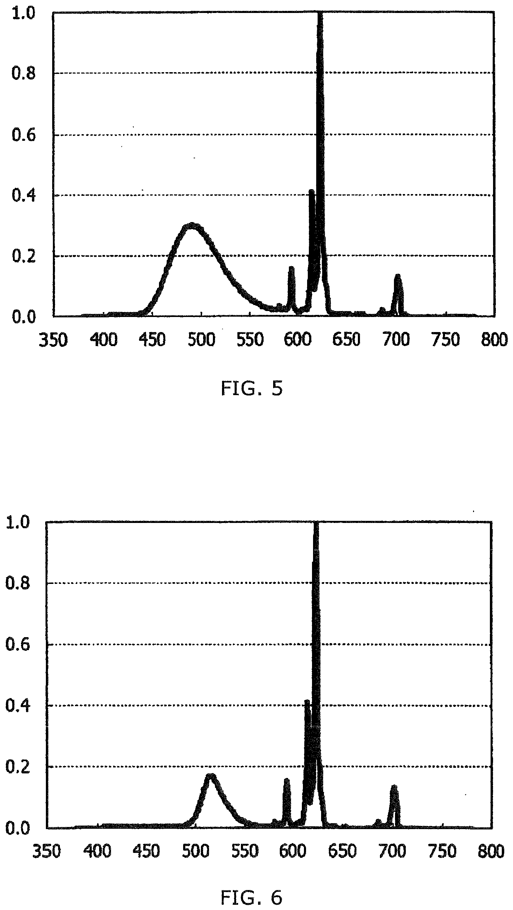

As an LED chip, one having an emission peak wavelength at 400 nm was provided and arranged on an alumina substrate. Then a mixed phosphor composed of blue green 1 and red 1 was provided. The average particle size of the respective phosphors was about 26 µm. The mixing ratio of the phosphors was, blue green 1: red 1 = 35:65 by weight (by mass). The mixed phosphor was mixed with a transparent resin (silicone resin) and the resulting mixture was coated on the LED chip provided with a transparent resin layer (thickness 0.5 mm) to obtain a white light source shown in Fig. 5. The thickness of the phosphor layer was 0.35 mm. Then the emission spectrum of the white light source was measured according to Example A1. In the emission spectrum of the white light source, the ratio (P3/P2) was 18%.

### (Example A4)

As an LED chip, one having an emission peak wavelength at 410 nm was provided and arranged on an alumina substrate. Then a mixed phosphor composed of green 1 and red 1 was provided. The average particle size of the respective phosphors was about 18 µm. The mixing ratio of the phosphors was, green 1: red 1 = 20:80 by weight (by mass). The mixed phosphor was mixed with a transparent resin (silicone resin) and the resulting mixture was coated on the LED chip provided with a transparent resin layer (thickness 0.3 mm) to obtain a white light source shown in Fig. 6. The thickness of the phosphor layer was 0.25 mm. Then the emission spectrum of the white light source was measured according to Example A1. In the emission spectrum of the white light source, the ratio (P3/P2) was 15%.

### (Example A5)

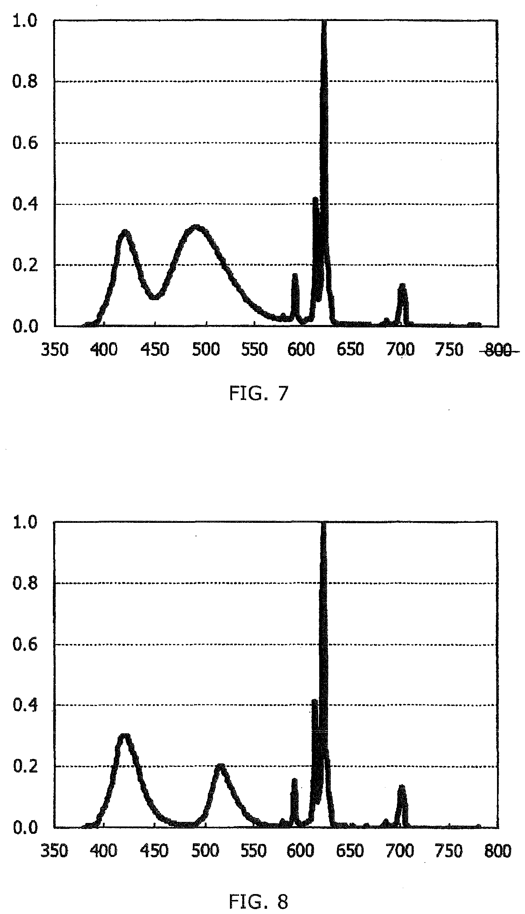

As an LED chip, one having an emission peak wavelength at 390 nm was provided and arranged on an alumina substrate. Then a mixed phosphor composed of blue 2, blue green1 and red 1 was provided. The average particle size of the respective phosphors was about 10 µm. The mixing ratio of the phosphors was, blue 2: blue green 1: red 1 = 20:25:55 by weight (by mass). The mixed phosphor was mixed with a transparent resin (silicone resin) and the resulting mixture was coated on the LED chip provided with a transparent resin layer (thickness 0.2 mm) to obtain a white light source shown in Fig. 7. The thickness of the phosphor layer was 0.1 mm. Then the emission spectrum of the white light source was measured according to Example A1. In the emission spectrum of the white light source, the ratio (P3/P2) was 19%.

### (Example A6)

As an LED chip, one having an emission peak wavelength at 390 nm was provided and arranged on an alumina substrate. Then a mixed phosphor composed of blue 2, green 1 and red 1 was provided. The average particle size of the respective phosphors was about 10 µm. The mixing ratio of the phosphors was, blue 2: green 1: red 1 = 30:15:55 by weight (by mass). The mixed phosphor was mixed with a transparent resin (silicone resin) and the resulting mixture was coated on the LED chip provided with a transparent resin layer (thickness 0.2 mm) to obtain a white light source shown in Fig. 8. The thickness of the phosphor layer was 0.12 mm. Then the emission spectrum of the white light source was measured according to Example A1. In the emission spectrum of the white light source, the ratio (P3/P2) was 18%.

### (Example A7)

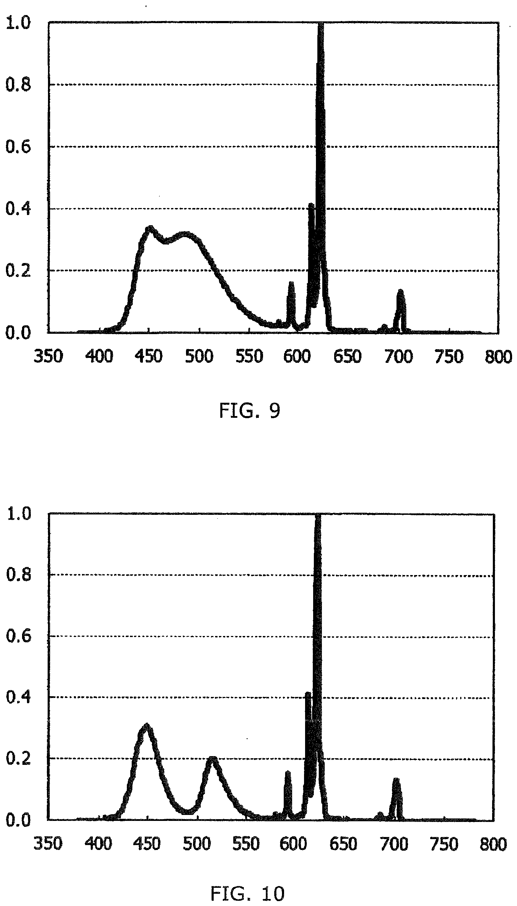

As an LED chip, one having an emission peak wavelength at 400 nm was provided and arranged on an alumina substrate. Then a mixed phosphor composed of blue 1, blue green 1 and red 1 was provided. The average particle size of the respective phosphors was about 15 µm. The mixing ratio of the phosphors was, blue 1: blue green 1: red 1 = 20:20:60 by weight (by mass). The mixed phosphor was mixed with a transparent resin (silicone resin) and the resulting mixture was coated on the LED chip provided with a transparent resin layer (thickness 0.3 mm) to obtain a white light source shown in Fig. 9. The thickness of the phosphor layer was 0.2 mm. Then the emission spectrum of the white light source was measured according to Example A1. In the emission spectrum of the white light source, the ratio (P3/P2) was 18%.

### (Example A8)

As an LED chip, one having an emission peak wavelength at 400 nm was provided and arranged on an alumina substrate. Then a mixed phosphor composed of blue 1, green 1 and red 1 was provided. The average particle size of the respective phosphors was about 17 µm. The mixing ratio of the phosphors was, blue 1: green 1: red 1 = 15:15:70 by weight (by mass). The mixed phosphor was mixed with a transparent resin (silicone resin) and the resulting mixture was coated on the LED chip provided with a transparent resin layer (thickness 0.3 mm) to obtain a white light source shown in Fig. 10. The thickness of the phosphor layer was 0.18 mm. Then the emission spectrum of the white light source was measured according to Example A1. In the emission spectrum of the white light source, the ratio (P3/P2) was 18%.

### (Example A9)

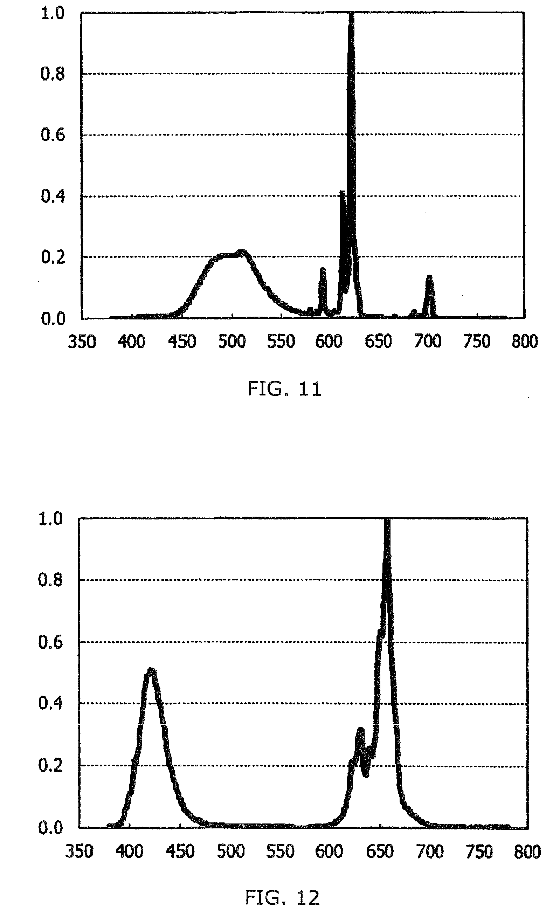

As an LED chip, one having an emission peak wavelength at 400 nm was provided and arranged on an alumina substrate. Then a mixed phosphor composed of blue green 1, green 1 and red 1 was provided. The average particle size of the respective phosphors was about 5 µm. The mixing ratio of the phosphors was, blue green 1: green 1: red 1 = 20:20:60 by weight (by mass). The mixed phosphor was mixed with a transparent resin (silicone resin) and the resulting mixture was coated on the LED chip provided with a transparent resin layer (thickness 0.1 mm) to obtain a white light source shown in Fig. 11. The thickness of the phosphor layer was 0.08 mm. Then the emission spectrum of the white light source was measured according to Example A1. In the emission spectrum of the white light source, the ratio (P3/P2) was 19%.

### (Example A10)

As an LED chip, one having an emission peak wavelength at 390 nm was provided and arranged on an alumina substrate. Then a mixed phosphor composed of blue 2 and red 2 was provided. The average particle size of the respective phosphors was about 8 µm. The mixing ratio of the phosphors was, blue 2: red 2 = 40:60 by weight (by mass). The mixed phosphor was mixed with a transparent resin (silicone resin) and the resulting mixture was coated on the LED chip provided with a transparent resin layer (thickness 0.15 mm) to obtain a white light source shown in Fig. 12. The thickness of the phosphor layer was 0.09 mm. Then the emission spectrum of the white light source was measured according to Example A1. In the emission spectrum of the white light source, the ratio (P3/P2) was 5%.

### (Example A11)

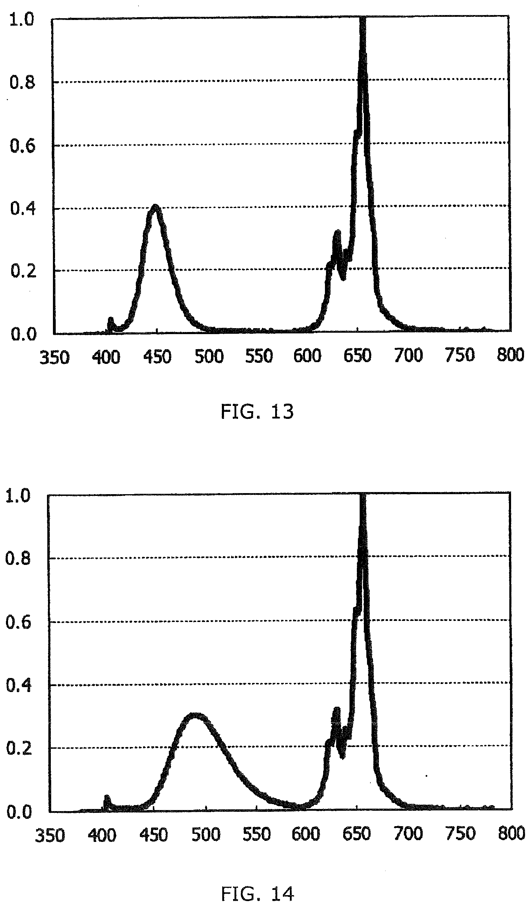

As an LED chip, one having an emission peak wavelength at 410 nm was provided and arranged on an alumina substrate. Then a mixed phosphor composed of blue 1 and red 2 was provided. The average particle size of the respective phosphors was about 20 µm. The mixing ratio of the phosphors was, blue 1: red 2 = 50:50 by weight (by mass). The mixed phosphor was mixed with a transparent resin (silicone resin) and the resulting mixture was coated on the LED chip provided with a transparent resin layer (thickness 0.4 mm) to obtain a white light source shown in Fig. 13. The thickness of the phosphor layer was 0.20 mm. Then the emission spectrum of the white light source was measured according to Example A1. In the emission spectrum of the white light source, the ratio (P3/P2) was 5%.

### (Example A12)

As an LED chip, one having an emission peak wavelength at 410 nm was provided and arranged on an alumina substrate. Then a mixed phosphor composed of blue green 1 and red 2 was provided. The average particle size of the respective phosphors was about 15 µm. The mixing ratio of the phosphors was, blue green 1: red 2 = 45:55 by weight (by mass). The mixed phosphor was mixed with a transparent resin (silicone resin) and the resulting mixture was coated on the LED chip provided with a transparent resin layer (thickness 0.2 mm) to obtain a white light source shown in Fig. 14. The thickness of the phosphor layer was 0.15 mm. Then the emission spectrum of the white light source was measured according to Example A1. In the emission spectrum of the white light source, the ratio (P3/P2) was 18%.

### (Example A13)

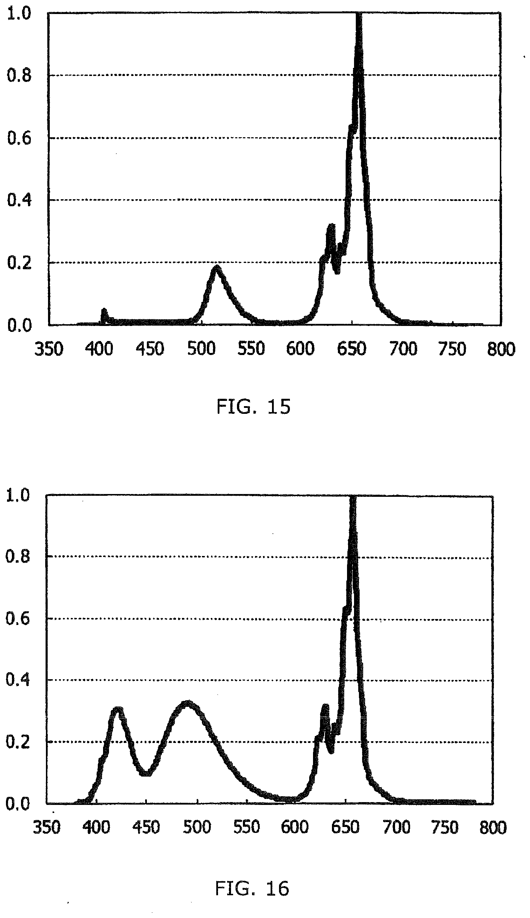

As an LED chip, one having an emission peak wavelength at 401 nm was provided and arranged on an alumina substrate. Then a mixed phosphor composed of green 1 and red 2 was provided. The average particle size of the respective phosphors was about 15 µm. The mixing ratio of the phosphors was, green 1: red 2 = 30:70 by weight (by mass). The mixed phosphor was mixed with a transparent resin (silicone resin) and the resulting mixture was coated on the LED chip provided with a transparent resin layer (thickness 0.3 mm) to obtain a white light source shown in Fig. 15. The thickness of the phosphor layer was 0.16 mm. Then the emission spectrum of the white light source was measured according to Example A1. In the emission spectrum of the white light source, the ratio (P3/P2) was 16%.

### (Example A14)

As an LED chip, one having an emission peak wavelength at 390 nm was provided and arranged on an alumina substrate. Then a mixed phosphor composed of blue 2, blue green 1 and red 2 was provided. The average particle size of the respective phosphors was about 12 µm. The mixing ratio of the phosphors was, blue 2: blue green 1: red 2 = 25:30:45 by weight (by mass). The mixed phosphor was mixed with a transparent resin (silicone resin) and the resulting mixture was coated on the LED chip provided with a transparent resin layer (thickness 0.3 mm) to obtain a white light source shown in Fig. 16. The thickness of the phosphor layer was 0.12 mm. Then the emission spectrum of the white light source was measured according to Example A1. In the emission spectrum of the white light source, the ratio (P3/P2) was 19%.

### (Example A15)

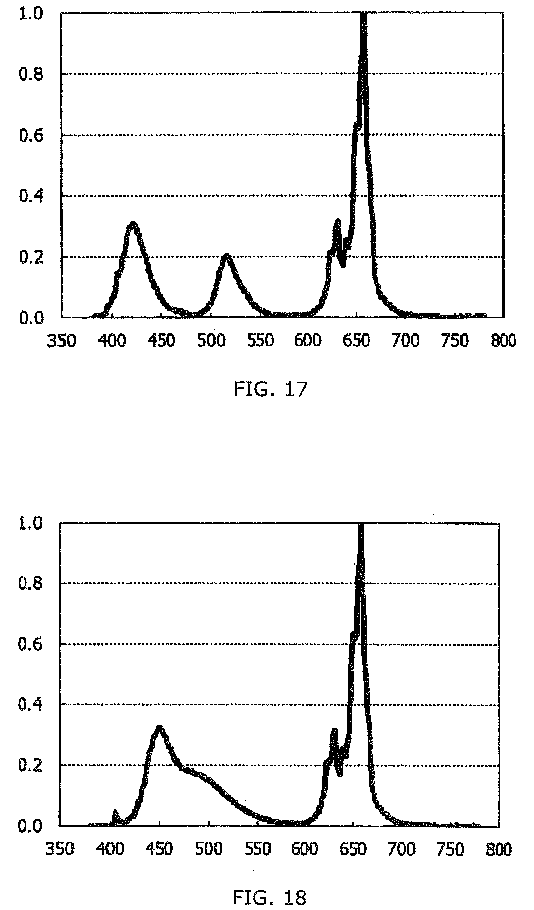

As an LED chip, one having an emission peak wavelength at 390 nm was provided and arranged on an alumina substrate. Then a mixed phosphor composed of blue 2, green 1 and red 2 was provided. The average particle size of the respective phosphors was about 11 µm. The mixing ratio of the phosphors was, blue 2: green 1: red 2 = 35:20:45 by weight (by mass). The mixed phosphor was mixed with a transparent resin (silicone resin) and the resulting mixture was coated on the LED chip provided with a transparent resin layer (thickness 0.2 mm) to obtain a white light source shown in Fig. 17. The thickness of the phosphor layer was 0.1 mm. Then the emission spectrum of the white light source was measured according to Example A1. In the emission spectrum of the white light source, the ratio (P3/P2) was 18%.

### (Example A16)

As an LED chip, one having an emission peak wavelength at 402 nm was provided and arranged on an alumina substrate. Then a mixed phosphor composed of blue 1, blue green 1 and red 2 was provided. The average particle size of the respective phosphors was about 9 µm. The mixing ratio of the phosphors was, blue 1: blue green 1: red 2 = 25:25:50 by weight (by mass). The mixed phosphor was mixed with a transparent resin (silicone resin) and the resulting mixture was coated on the LED chip provided with a transparent resin layer (thickness 0.1 mm) to obtain a white light source shown in Fig. 18. The thickness of the phosphor layer was 0.1 mm. Then the emission spectrum of the white light source was measured according to Example A1. In the emission spectrum of the white light source, the ratio (P3/P2) was 9%.

### (Example A17)

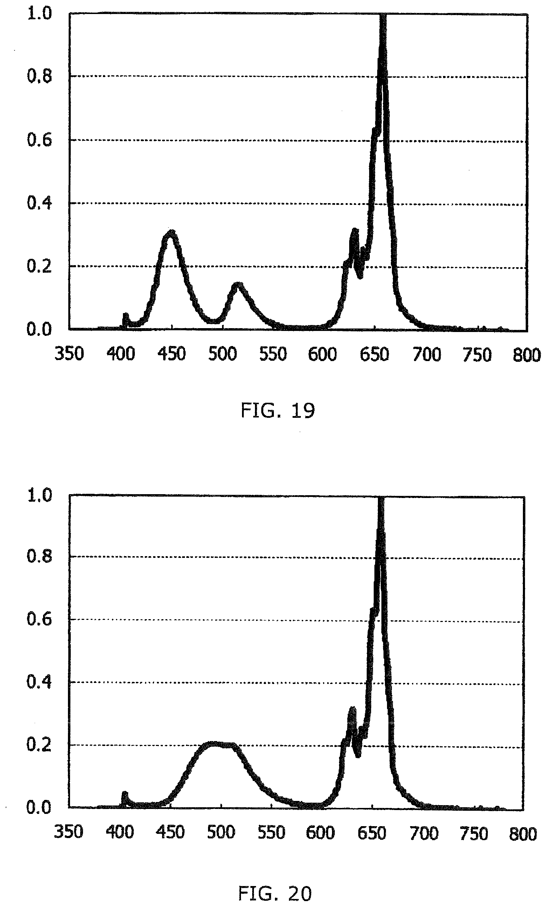

As an LED chip, one having an emission peak wavelength at 400 nm was provided and arranged on an alumina substrate. Then a mixed phosphor composed of blue 1, green 1 and red 2 was provided. The average particle size of the respective phosphors was about 16 µm. The mixing ratio of the phosphors was, blue 1: green 1: red 2 = 20:20:60 by weight (by mass). The mixed phosphor was mixed with a transparent resin (silicone resin) and the resulting mixture was coated on the LED chip provided with a transparent resin layer (thickness 0.3 mm) to obtain a white light source shown in Fig. 19. The thickness of the phosphor layer was 0.18 mm. Then the emission spectrum of the white light source was measured according to Example A1. In the emission spectrum of the white light source, the ratio (P3/P2) was 12%.

### (Example A18)

As an LED chip, one having an emission peak wavelength at 400 nm was provided and arranged on an alumina substrate. Then a mixed phosphor composed of blue green 1, green 1 and red 2 was provided. The average particle size of the respective phosphors was about 18 µm. The mixing ratio of the phosphors was, blue green 1: green 1: red 2 = 25:25:50 by weight (by mass). The mixed phosphor was mixed with a transparent resin (silicone resin) and the resulting mixture was coated on the LED chip provided with a transparent resin layer (thickness 0.4 mm) to obtain a white light source shown in Fig. 20. The thickness of the phosphor layer was 0.2 mm. Then the emission spectrum of the white light source was measured according to Example A1. In the emission spectrum of the white light source, the ratio (P3/P2) was 17%.

### (Comparative Example A1)

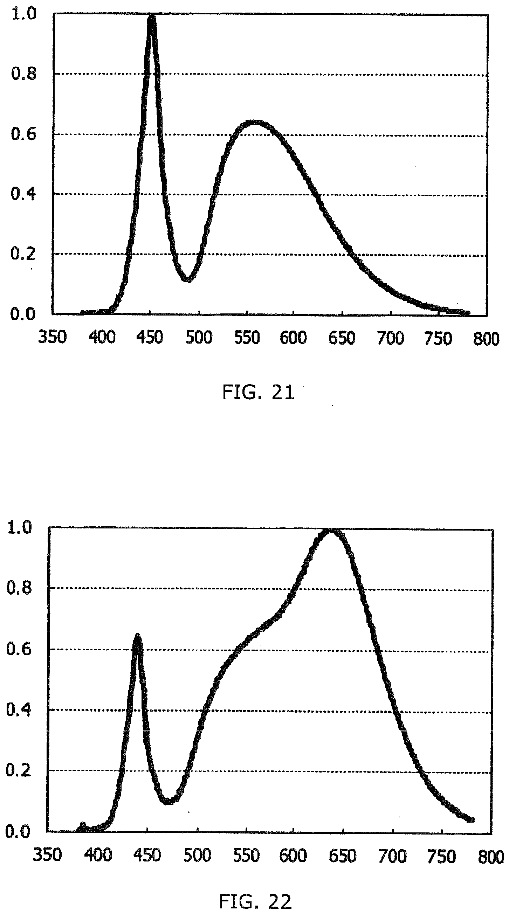

A commercially available white light source, in which a blue LED is combined with a yellow phosphor and a red phosphor, was provided as Comparative Example A1. The chemical compositions or the like of the blue LED or phosphors were not clear, but the emission spectrum of the white light source was measured according to Example A to obtain the data in a shape shown in Fig. 21. In the white light source of Comparative Example A1, the spectrum shape was not adjusted like the present invention and the ratio (P3/P2) was 64%.

### (Comparative Example A2)

A different type of a commercially available white light source from one in Comparative Example A1, in which a blue LED is combined with a yellow phosphor and a red phosphor, was provided as Comparative Example A2. The chemical compositions or the like of the blue LED or phosphors were not clear, but the emission spectrum of the white light source was measured according to Example A1 to obtain the data in a shape shown in Fig. 22. In the white light source of Comparative Example A2, the spectrum shape was not adjusted like the present invention and the ratio (P3/P2) was 71%.

### (Examples A19 to A23)

The effect of the film thickness of a phosphor film, presence or absence of a transparent resin layer and the film thickness of the transparent resin layer on the brightness of a white light source was verified. Using the same LED chip and mixed phosphor film as used in Example A1, white light sources with the film thickness of the phosphor films and the transparent resin layers thereof altered were prepared. The resulting light sources were connected to a power supply and electric power of 8W was applied to compare the brightness of each light source. The results are shown in Table 1.

**[Table 1]**

| | Combination of phosphors | Film thickness of the phosphor film (mm) | Film thickness of the transparent resin layer (mm) | Brightness of the light source (lm/w) |
|---|---|---|---|---|
| Example A19 | The light source of Example A1 | 0.09 | 0.3 | 27 |
| Example A20 | | 0.2 | 0.3 | 33 |
| Example A21 | | 0.7 | 0.3 | 30 |
| Example A22 | | 0.2 | 1.5 | 31 |
| Example A23 | | 0.2 | 0 | 28 |

With reference to the results shown in Table 1, in respect to the effect of the presence or absence of a transparent resin layer, the comparison of Example A20 and Example A23 indicates that the light source with a transparent resin layer formed therein was brighter, and it is obvious that a transparent resin layer has an advantage in the improvement of the brightness of a light source. However, even when the transparent resin layer is formed, if the film thickness of the transparent resin layer is too thick as in Example A22, though the energy loss due to an LED chip is reduced, the influence of the light absorption by the transparent resin layer itself becomes bigger and the brightness reduces depending on the surplus thickness in relative to the appropriate film thickness. As in Example A19, if the film thickness of a phosphor layer is as thin as the appropriate value or thinner than the appropriate value, a bright light source cannot be obtained because the LED light which is directed towards the phosphor layer is not absorbed on the phosphor layer sufficiently and leaks outside the light source. As in Example A20, if the film thickness of the phosphor layer is the appropriate value or more, a light source which emits bright light can be obtained; however, if the film thickness of the phosphor layer is excessively thick as in Example A21, though the loss of LED light does not occur, the emission by the phosphor layer itself is not taken outside the phosphor layer and the brightness starts decreasing.

A use test was performed on the light source obtained as described above in an actual medical field to verify if it was available as a white light source for medicine that exhibits the effect of the present invention. The light sources used in the verification test are as follows. For white light sources prepared in Examples A1 to A18 and Comparative Examples A1 and A2, 10 white light sources were provided from each Example and Comparative Example to prepare white light source systems. A light source system for medicine has 10 light sources for medicine arranged at 2 mm intervals on a single mounting substrate. The light source system for medicine is bulb-shaped with a power supply and a predetermined cover attached.

The resulting light source systems for medicine then were used to perform an verification test if the light source systems for medicine were capable of illuminating an arm of the human body and distinguishing blood vessels from the skin. The verification test was performed with the cooperation from 50 patients who came to hospital and five nurses. Among patients who cooperated were 10 Japanese patients from each age group of 15 or less, 16 to 35, 36 to 65 and 65 or more and 10 Western white patients aged 36 to 65. The selection of 10 patients from each age group was made randomly, but it was noted that the numbers of males and females would be almost the same. Five trained nurses with 10 year experience cooperated. The test was performed as described below. In a room which was illuminated by a general fluorescent light for household, a table for injection was provided and a desk lamp was provided thereon, and the lights of the light source systems for medicine were irradiated on the arm of a patient to observe the arm. For each light source system for medicine, five nurses observed the arms of 50 patients in a random order and compared the discernibility of blood vessels. The evaluated ranking had three following stages and was retained as a primary record.
A: The use of the LED bulb makes an obvious difference in colors of blood vessels and the skin and allows for easy distinction
B: The use of the LED bulb allows for distinction between blood vessels and the skin as usual
C: The use of an LED bulb does not make it easy to distinguish blood vessels in the skin

The results of the primary record was then reevaluated and the final ranking was made as shown below. The criteria for the final evaluation had three stages as follows.
⊚: A light source in which 90% or more of the evaluation results are As and less than 1% are Cs.
∘: A light source in which 99% or more of the evaluation results are Bs
×: A light source in which 1% or more the evaluation results are Cs.

The results of the visibility evaluation are shown in Table 2 as well as the light source types.

**[Table 2]**

| Liqht sources used | | | | | | | (P3/P2) (%) | Visibility evaluation |
|---|---|---|---|---|---|---|---|---|
| Light source | Mixing ratio of phosphors (mass %) | | | | | | | |
| | Blue 1 | Blue 2 | Blue green 1 | Green 1 | Red 1 | Red 2 | | |
| Example A1 | - | 30 | - | - | 70 | - | 2 | ⊚ |
| Example A2 | 40 | - | - | - | 60 | - | 2 | ⊚ |
| Example A3 | - | - | 35 | - | 65 | - | 18 | ○ |
| Example A4 | - | - | - | 20 | 80 | - | 15 | ⊚ |
| Example A5 | - | 20 | 25 | - | 55 | - | 19 | ○ |
| Example A6 | - | 30 | - | 15 | 55 | - | 18 | ○ |
| Example A7 | 20 | - | 20 | - | 60 | - | 18 | ○ |
| Example A8 | 15 | - | - | 15 | 70 | - | 18 | ○ |
| Example A9 | - | - | 20 | 20 | 60 | - | 19 | ○ |
| Example A10 | - | 40 | - | - | - | 60 | 5 | ⊚ |
| Example A11 | 50 | - | - | - | - | 50 | 5 | ⊚ |
| Example A12 | - | - | 45 | - | - | 55 | 18 | ○ |
| Example A13 | - | - | - | 30 | - | 70 | 16 | ○ |
| Example A14 | - | 25 | 30 | - | - | 45 | 19 | ○ |
| Example A15 | - | 35 | - | 20 | - | 45 | 18 | ○ |
| Example A16 | 25 | - | 25 | - | - | 50 | 9 | ⊚ |
| Example A17 | 20 | - | - | 20 | - | 60 | 12 | ⊚ |
| Example A18 | - | - | 25 | 25 | - | 50 | 17 | ○ |
| Comparative Example A1 | Commercially available light sources were used | | | | | | 64 | × |
| Comparative Example A2 | | | | | | | 71 | × |

### (Examples B1 to B7, Comparative Example B1)

An LED chip was arranged on an alumina substrate. A transparent resin layer (thickness 0.2 mm) composed of a silicone resin was provided on the LED chip. Then, a mixed phosphor composed of blue 2 and red 1 was provided. The average particle size of each phosphor was 18 µm. The mixed phosphor was mixed with a silicone resin and the resulting mixture was coated on the transparent resin layer and cured to provide a phosphor layer (thickness 0.03 mm). In this way, light sources for medicine according to Examples B1 to 7 and Comparative Example B1 were prepared. The emission peak wavelengths of LED chips and the mixing ratios (mass ratios) in which the total of blue 2 and red 1 phosphors is 100 parts by mass are shown in Table 3.

### (Comparative Example B2)

A commercially available white light source in which a blue LED (emission peak wavelength 450 nm) was used with a cerium-activated aluminate yttrium phosphor (YAG:Ce phosphor) was used.

**[Table 3]**

| | Emission peak wavelength of the LED (nm) | Mixing ratio of phosphors (parts by mass) | |
|---|---|---|---|
| | | Blue 2 | Red 1 |
| Example B1 | 405 | 10 | 90 |
| Example B2 | 405 | 30 | 70 |
| Example B3 | 405 | 45 | 55 |
| Example B4 | 405 | 55 | 45 |
| Example B5 | 405 | 60 | 40 |
| Example B6 | 405 | 70 | 30 |
| Example B7 | 405 | 80 | 20 |
| Comparative Example B1 | 405 | 2 | 98 |
| Comparative Example B2 | 450 | Yellow phosphor alone | |

The emission spectra of the white light sources for medicine according to Examples and Comparative Examples were measured. The measurement of the emission spectra was performed by a total luminous flux measuring apparatus equipped with an integrating sphere according to JIS-C-8152.

From the results of the emission spectra, the maximum intensity of the emission spectrum within a wavelength region from 370 nm to 470 nm (P1), the maximum intensity of the emission spectrum within a wavelength region from 600 nm to 780 nm (P2), the maximum intensity of the emission spectrum within a wavelength region from 520 nm to 580 nm (P3), the area of the emission spectrum within a wavelength region from 370 nm to 780 nm (S1), the area of the emission spectrum within a wavelength region from 471 to 599 nm (S2) were elicited. The ratio (P1/P2), the ratio (S2/S1) and the ratio (P3/P2) are shown in Table 4. Here, examples B1, B6, and B7 do not fall under claim 1.

**[Table 4]**

| | Ratio(P1/P2) | Ratio(S2/S1) | Ratio(P3/P2) |
|---|---|---|---|
| Example B1 | 0.3 | 0.10 | 0 |
| Example B2 | 0.5 | 0.10 | 0 |
| Example B3 | 0.9 | 0.10 | 0 |
| Example B4 | 1.0 | 0.10 | 0 |
| Example B5 | 1.2 | 0.10 | 0 |
| Example B6 | 1.4 | 0.10 | 0 |
| Example B7 | 1.5 | 0.10 | 0 |
| Comparative Example B1 | 0.1 | 0.10 | 0 |
| Comparative Example B2 | 1.91 | 0.51 | 1.23 |

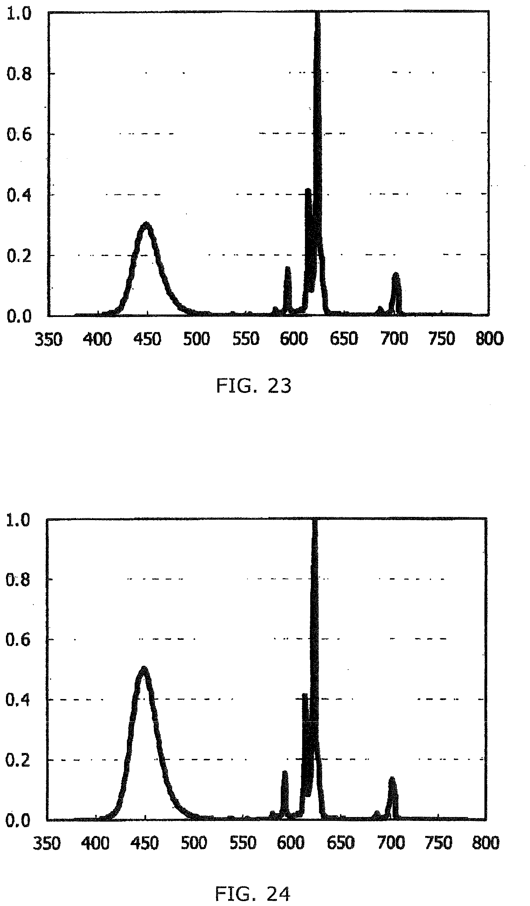

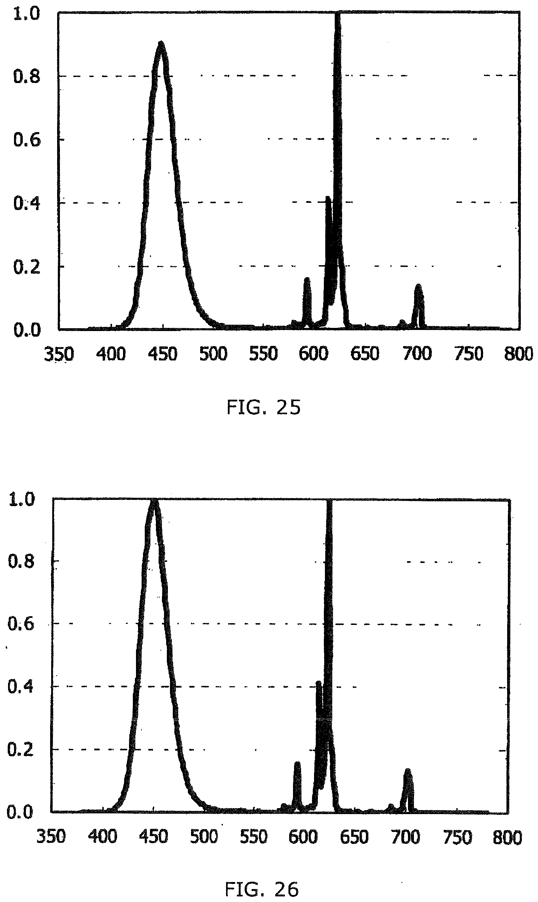

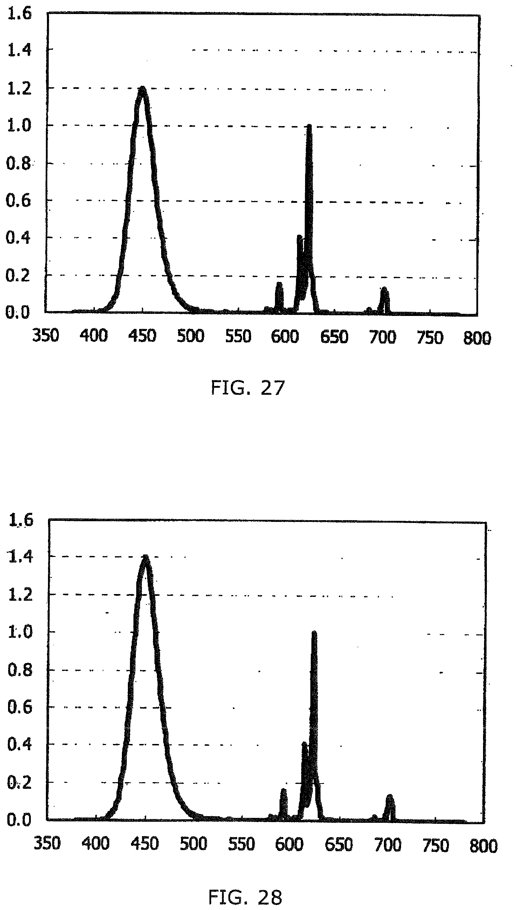

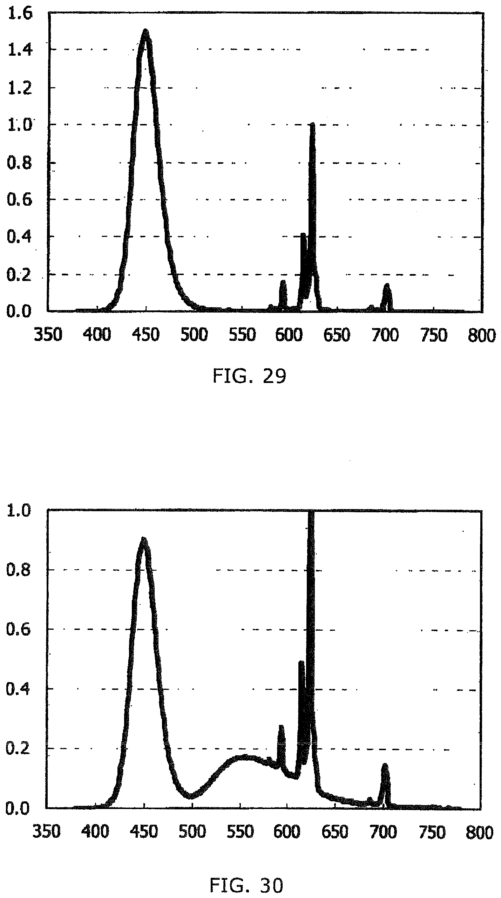

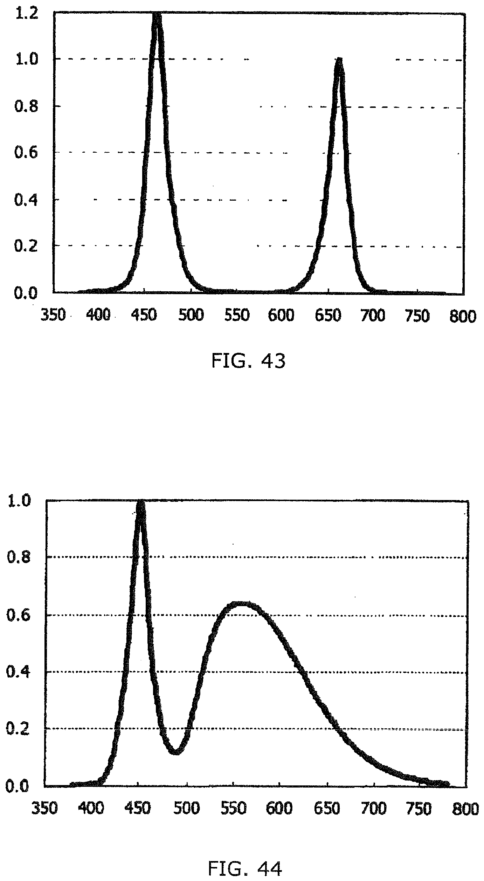

As seen in the table, Comparative Example B1 with a small mixing ratio of a blue phosphor had the ratio (P1/P2) of 0.1, which was less than the ratio of the present embodiment, 0.3. The emission spectra are shown in Fig. 23 (Example B1), Fig. 24 (Example B2), Fig. 25 (Example B3), Fig. 26 (Example B4), Fig. 27 (Example B5), Fig. 28 (Example B6), Fig. 29 (Example B7) and Fig. 44 (Comparative Example B2). In each emission spectrum, the abscissa indicates the wavelength and the ordinate indicates the emission intensity. As seen in the figures, the light sources for medicine according to Examples had peaks within wavelength regions from 370 nm to 470 nm and 600 nm to 780 nm. On the other hand, Comparative Example 2 did not have a peak within a wavelength region from 600 nm to 780 nm. The ratio (P3/P2) of Comparative Example B2 was elicited wherein P2 was the emission intensity of 600 nm.

### (Examples B8 to B13)

An LED chip was arranged on an alumina substrate. A transparent resin layer (thickness 0.3 mm) composed of a silicone resin was provided on the LED chip. Then, a mixed phosphor composed of blue 1, yellow 1 and red 1 was provided. The average particle size of each phosphor was 22 µm. The mixed phosphor was mixed with a silicone resin and the resulting mixture was coated on the transparent resin layer and cured to provide a phosphor layer (thickness 0.06 mm). In this way, light sources for medicine according to Examples B8 to B13 were prepared. The emission peak wavelengths of LED chips and the mixing ratios (mass ratios) in which the total of blue 1, yellow 1 and red 1 phosphors is 100 parts by mass are shown in Table 5.

**[Table 5]**

| | Emission peak wavelength of the LED (nm) | Mixing ratio of phosphors (parts by mass) | | |
|---|---|---|---|---|
| | | Blue 1 | Yellow 1 | Red 1 |
| Example B8 | 395 | 35 | 23 | 42 |
| Example B9 | 395 | 38 | 17 | 46 |
| Example B10 | 395 | 41 | 9 | 50 |
| Example B11 | 395 | 43 | 5 | 52 |
| Example B12 | 395 | 45 | 0 | 55 |
| Example B13 | 395 | 30 | 0 | 70 |

The emission spectra of the white light sources for medicine according to Examples were measured in the same way as in Example B1. The results are shown in Table 6.

**[Table 6]**

| | Ratio (P1/P2) | Ratio (S2/S1) | Ratio (P3/P2) |
|---|---|---|---|
| Example B8 | 0.9 | 0.30 | 0.18 |
| Example B9 | 0.9 | 0.25 | 0.10 |
| Example B10 | 0.9 | 0.20 | 0.08 |
| Example B11 | 0.9 | 0.15 | 0.05 |
| Example B12 | 0.9 | 0.10 | 0 |
| Example B13 | 0.5 | 0.05 | 0 |

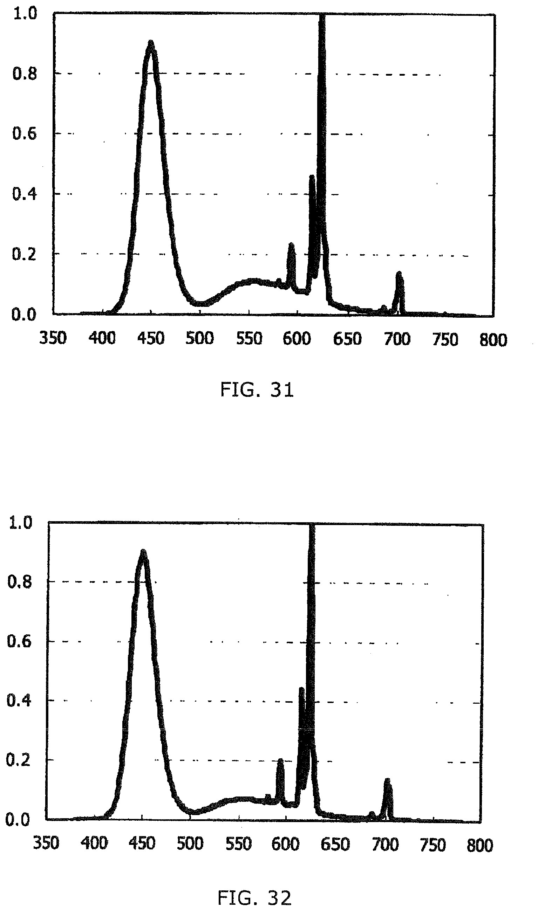

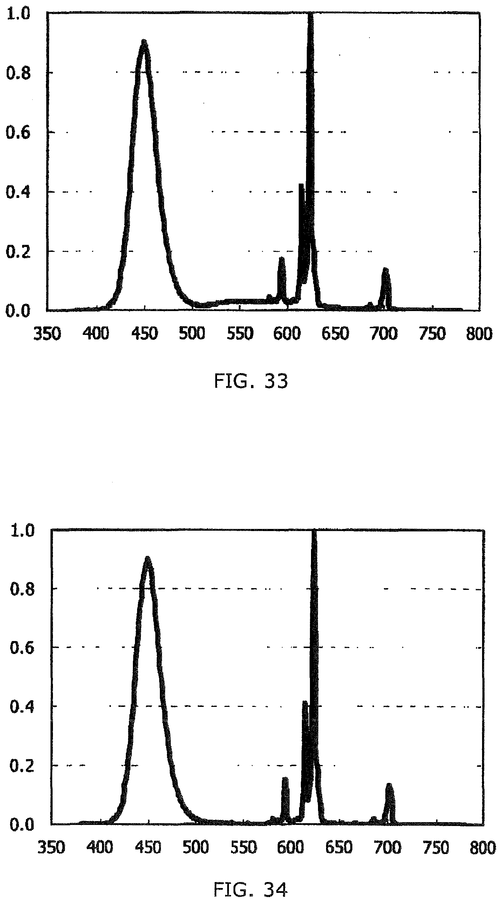

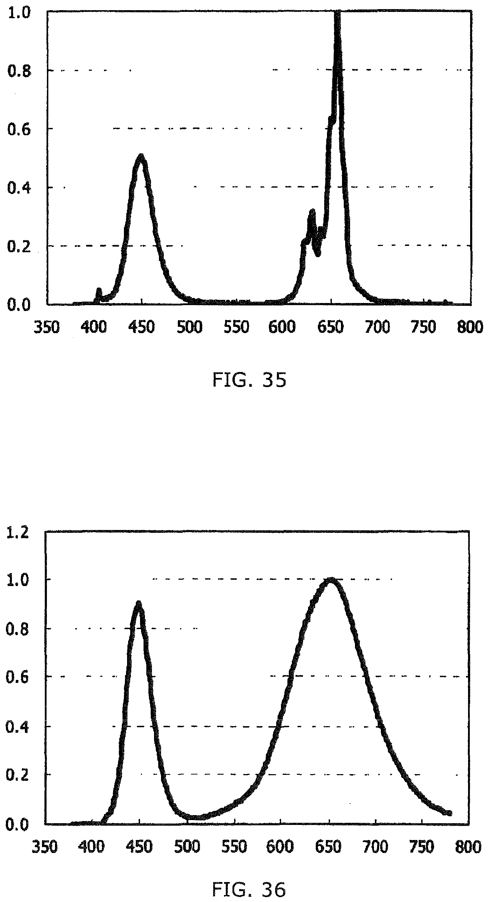

As seen in the table, obtained emission spectra of light sources for medicine were applicable to the present embodiment. The emission spectra are shown in Fig. 30 (Example B8), Fig. 31 (Example B9), Fig. 32 (Example B10), Fig. 33 (Example B11), Fig. 34 (Example B12) and Fig. 35 (Example B13). In each emission spectrum, the abscissa indicates the wavelength and the ordinate indicates the emission intensity. As seen in the figures, the light sources for medicine according to Examples had peaks within wavelength regions from 370 nm to 470 nm and 600 nm to 780 nm.

### (Examples B14 to B18)

An LED chip was arranged on an alumina substrate. A transparent resin layer (thickness 0.3 mm) composed of a silicone resin was provided on the LED chip. Then, a mixed phosphor composed of blue 1, green 1 and red 3 was provided. The average particle size of each phosphor was 20 µm. The mixed phosphor was mixed with a silicone resin and the resulting mixture was coated on the transparent resin layer and cured to provide a phosphor layer (thickness 0.07 mm). In this way, light sources for medicine according to Examples B14 to B18 were prepared. The emission peak wavelengths of LED chips and the mixing ratios (mass ratios) in which the total of blue 1, yellow 1 and red 3 phosphors is 100 parts by mass are shown in Table 3.

**[Table 7]**

| | Emission peak wavelength of the LED (nm) | Mixing ratio of phosphors (parts by mass) | | |
|---|---|---|---|---|
| | | Blue 1 | Yellow 1 | Red 3 |
| Example B14 | 410 | 40 | 0 | 60 |
| Example B15 | 410 | 36 | 9 | 55 |
| Example B16 | 410 | 33 | 17 | 50 |
| Example B17 | 410 | 31 | 23 | 46 |
| Example B18 | 410 | 29 | 28 | 43 |

The emission spectra of the white light sources for medicine according to Examples were measured in the same way as in Example B1. The results are shown in Table 8.

**[Table 8]**

| | Ratio (P1/P2) | Ratio (S2/S1) | Ratio (P3/P2) |
|---|---|---|---|
| Example B14 | 0.9 | 0.11 | 0.20 |
| Example B15 | 0.9 | 0.13 | 0.25 |
| Example B16 | 0.9 | 0.16 | 0.30 |
| Example B17 | 0.9 | 0.19 | 0.35 |
| Example B18 | 0.9 | 0.21 | 0.40 |

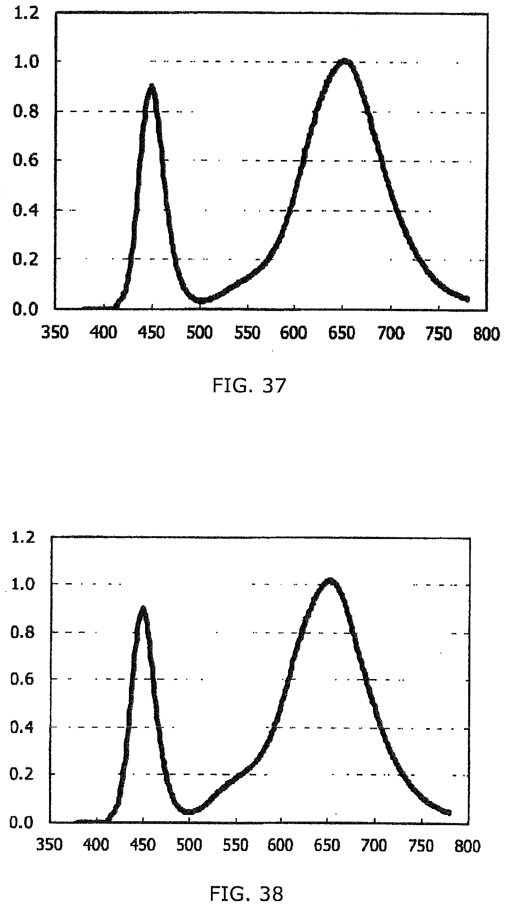

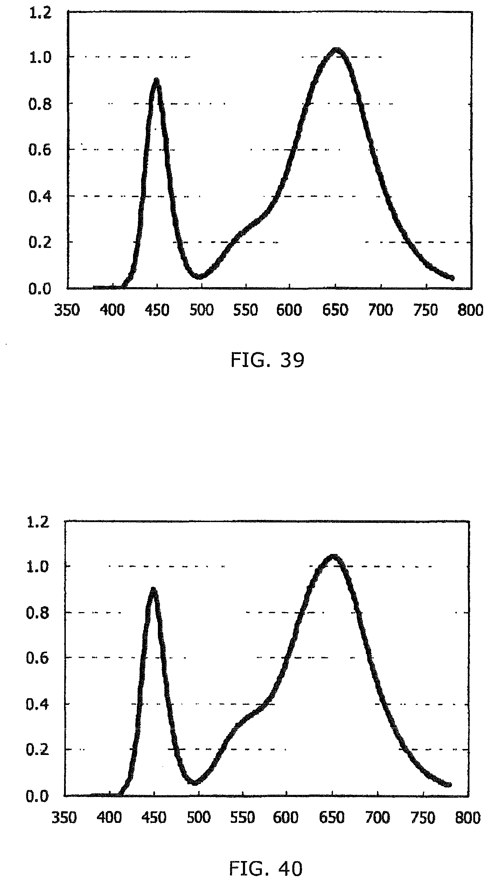

As seen in the table, obtained emission spectra of light sources for medicine were applicable to the present embodiment. The emission spectra are shown in Fig. 36 (Example B14), Fig. 37 (Example B15), Fig. 38 (Example B16), Fig. 39 (Example B17) and Fig. 40 (Example B18). In each emission spectrum, the abscissa indicates the wavelength and the ordinate indicates the emission intensity. As seen in the figures, the light sources for medicine according to Examples had peaks within wavelength regions from 370 nm to 470 nm and 600 nm to 780 nm.

### (Examples B19 to B21)

A blue LED (Emission peak wavelength 460 nm) and a red LED (emission peak wavelength 660 nm) were arranged on an alumina substrate, each spaced 2 mm apart. Light sources for medicine with different emission spectra were prepared by changing the current values of the blue LEDs and the red LEDs. The emission spectra were measured in the same way as in Example B1. The results are shown in Table 9.

**[Table 9]**

| | Ratio (P1/P2) | Ratio (S2/S1) | Ratio (P3/P2) |
|---|---|---|---|
| Example B19 | 0.9 | 0.14 | 0 |
| Example B20 | 1.0 | 0.15 | 0 |
| Example B21 | 1.2 | 0.16 | 0 |

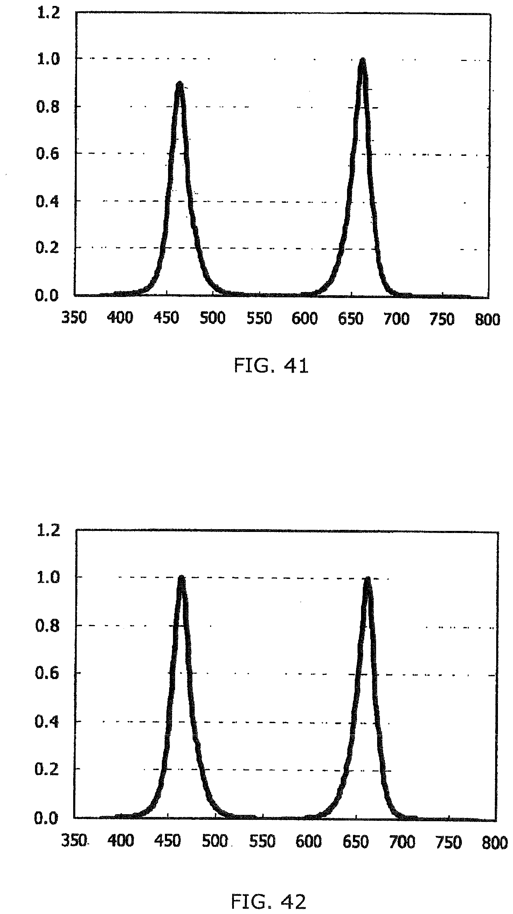

As seen in the table, obtained emission spectra of light sources for medicine were applicable to the present embodiment. The emission spectra are shown in Fig. 41 (Example B19), Fig. 42 (Example B20) and Fig. 43 (Example B21). In each emission spectrum, the abscissa indicates the wavelength and the ordinate indicates the emission intensity. As seen in the figures, the light sources for medicine according to Examples had peaks within wavelength regions from 370 nm to 470 nm and 600 nm to 780 nm.

For light sources according to Examples B1 to A21 and Comparative Examples B1 and B2, 10 light sources from each Example and Comparative Example were provided to prepare white light source systems. A light source system for medicine has 10 light sources for medicine arranged at 2 mm intervals on a single mounting substrate. The light source system for medicine is bulb-shaped with a power supply and a predetermined cover attached.

Then, the resulting light source systems for medicines were measured for the visibility of blood vessels and the presence or absence of emission glare. For the visibility of blood vessels, a verification test was performed to see if the light source systems for medicine were capable of illuminating an arm of the human body and distinguishing blood vessels from the skin. The verification test was performed with the cooperation from 50 patients who came to hospital and five nurses. Among patients who cooperated were 10 Japanese patients from each age group of 20 to 30, 31 to 40, 41 to 50, 51 to 60 and 61 or more. The selection of 10 patients from each age group was made randomly, but it was noted that the numbers of males and females would be almost the same. As for nurses, five trained nurses with 10 year experience cooperated. The test was performed as described below. In a room which was illuminated by a general fluorescent light for household, a table for injection was provided and a desk lamp was provided thereon, and the lights of the light source systems for medicine were irradiated on the arm of a patient to observe the arm. For each light source system for medicine, five nurses observed the arms of 50 patients in a random order and compared the discernibility of blood vessels. The evaluation method was the similar as for the light source system of Example A1.

In addition, 100 patients chosen arbitrarily answered a questionnaire asking if the light felt uncomfortable or not, e.g., too bright, to measure the glare. As a result, ⊚ is when 20% of people or less felt uncomfortable; o is when 21 to 40% of people felt uncomfortable; and × is when 41% of people or more felt uncomfortable.

The results are shown in Table 10.

**[Table 10]**

| Light sources | Visibility of blood vessels | Glare |
|---|---|---|
| Example B1 | ○ | ⊚ |
| Example B2 | ○ | ⊚ |
| Example B3 | ⊚ | ⊚ |
| Example B4 | ⊚ | ⊚ |
| Example B5 | ⊚ | ⊚ |
| Example B6 | ⊚ | ⊚ |
| Example B7 | ○ | ○ |
| Example B8 | ○ | ⊚ |
| Example B9 | ○ | ⊚ |
| Example B10 | ⊚ | ⊚ |
| Example B11 | ⊚ | ⊚ |
| Example B12 | ⊚ | ⊚ |
| Example B13 | ○ | ⊚ |
| Example B14 | ⊚ | ⊚ |
| Example B15 | ⊚ | ⊚ |
| Example B16 | ○ | ⊚ |
| Example B17 | ○ | ⊚ |
| Example B18 | ○ | ⊚ |
| Example B19 | ⊚ | ○ |
| Example B20 | ⊚ | ○ |
| Example B21 | ⊚ | ○ |
| Comparative Example B1 | × | × |
| Comparative Example B2 | × | × |

As seen in the table, the light source systems according to Examples had great visibility of blood vessels and reduced glare. Especially, those with the ratio (P1/P2) of 0.5 to 1.2, the ratio (S2/S1) of 0.25 to 0.05 and the ratio (P3/P2) of 0.3 or less had more improved characteristics.

Accordingly, a handy and convenient light system for medicine can be structured by unitizing light sources for medicine according to Examples and a general lamp (LED lamp) in an switchable way and switching the light sources between the case of performing a blood vessel identification operation and the case of performing a normal operation.

Some embodiments of the present invention were exemplified above, but these embodiments are presented as examples and are not intended to limit the scope of the invention. The scope of the invention is defined by the claims.

### Explanation of References

- 1: WHITE LIGHT SOURCE
- 2: SUBSTRATE
- 3: LED CHIP
- 4: TRANSPARENT RESIN LAYER
- 5: PHOSPHOR LAYER
- 6: LIGHT SOURCE SYSTEM FOR MEDICINE
- 7: MOUNTING SUBSTRATE

## Claims

1. A white light source for medicine which is to be used for visually observing a blood vessel in skin, the light source having an emission spectrum, wherein the white light source comprises an LED chip having an emission peak within a wavelength region from 370 nm to 480 nm; and a phosphor layer which is excited by a primary light irradiated by the LED chip and emits a secondary light within visible light region, and wherein, in the emission spectrum, a P3 to P2 ratio (P3/P2) is 0.4 or less, wherein P3 is the maximum intensity of the emission spectrum within a wavelength region from 520 nm to 580 nm, and P2 is the maximum intensity of the emission spectrum within a wavelength region from 600 nm to 780 nm,
wherein a P1 to P2 ratio (P1/P2) is 0.5 to 1.2, wherein P1 is the maximum intensity of the emission spectrum within a wavelength region from 370 nm to 470 nm.

2. The white light source for medicine according to claim 1, wherein the ratio (P3/P2) is 15% or less.

3. The light source for medicine according to claim 1 or 2, wherein an S2 to S1 ratio (S2/S1) is 0.3 or less (including zero), wherein S1 is the area of the emission spectrum within a wavelength region from 370 nm to 780 nm, and S2 is the area of the emission spectrum within a wavelength region from 471 nm to 599 nm.

4. The light source for medicine according to one of the preceding claims, comprising a plurality of LED chips and having a structure wherein the phosphor layer covers a circumference of the LED chips.

5. The light source for medicine according to one of the preceding claims, comprising a transparent resin layer between the LED chip and the phosphor layer.

6. The light source for medicine according to claim 5, wherein the phosphor layer has a film thickness of at least 0.07 mm or more, and the transparent resin layer has a film thickness of 0.1 to 1.0 mm.

7. Fluorescent lamp using the light source for medicine according to claim 1 or 2.

8. A light source system for medicine, comprising a plurality of the light sources for medicine according to any one of claims 1 to 6.

9. A light source system for medicine, comprising a first light source which is the light source for medicine according to any one of claims 1 to 6, and a second white light source having an emission spectrum different from that of the first light source.

10. The light source system for medicine according to claim 9, wherein lighting of the first light source and the second light source can be switched.

## Patentansprüche

1. Weißlichtquelle für die Medizin, die zur visuellen Beobachtung eines Blutgefäßes in der Haut zu verwenden ist, wobei die Lichtquelle ein Emissionsspektrum aufweist, wobei die Weißlichtquelle einen LED-Chip mit einem Emissionspeak innerhalb eines Wellenlängenbereichs von 370 nm bis 480 nm umfasst; und eine Leuchtstoffschicht, die durch ein von dem LED-Chip eingestrahltes Primärlicht angeregt wird und ein Sekundärlicht innerhalb des sichtbaren Lichtbereichs emittiert, und wobei,
im Emissionsspektrum ein Verhältnis von P3 zu P2 (P3/P2) 0,4 oder weniger beträgt, wobei P3 die maximale Intensität des Emissionsspektrums innerhalb eines Wellenlängenbereichs von 520 nm bis 580 nm ist und P2 die maximale Intensität des Emissionsspektrums innerhalb eines Wellenlängenbereichs von 600 nm bis 780 nm ist,
wobei ein Verhältnis von P1 zu P2 (P1/P2) 0,5 bis 1,2 beträgt, wobei P1 die maximale Intensität des Emissionsspektrums innerhalb eines Wellenlängenbereichs von 370 nm bis 470 nm ist.

2. Weißlichtquelle für die Medizin nach Anspruch 1, wobei das Verhältnis (P3/P2) 15 % oder weniger beträgt.

3. Lichtquelle für die Medizin nach Anspruch 1 oder 2, wobei ein S2/S1-Verhältnis (S2/S1) 0,3 oder weniger (einschließlich Null) ist, wobei S1 die Fläche des Emissionsspektrums innerhalb eines Wellenlängenbereichs von 370 nm bis 780 nm ist und S2 die Fläche des Emissionsspektrums innerhalb eines Wellenlängenbereichs von 471 nm bis 599 nm ist.

4. Lichtquelle für die Medizin nach einem der vorstehenden Ansprüche, die eine Vielzahl von LED-Chips umfasst und eine Struktur aufweist, bei der die Leuchtstoffschicht einen Umfang der LED-Chips bedeckt.

5. Lichtquelle für die Medizin nach einem der vorstehenden Ansprüche, umfassend eine transparente Harzschicht zwischen dem LED-Chip und der Leuchtstoffschicht.

6. Lichtquelle für die Medizin nach Anspruch 5, wobei die Leuchtstoffschicht eine Filmdicke von mindestens 0,07 mm oder mehr und die transparente Harzschicht eine Filmdicke von 0,1 bis 1,0 mm aufweist.

7. Fluoreszenzlampe mit der Lichtquelle für die Medizin nach Anspruch 1 oder 2.

8. Lichtquellensystem für die Medizin, umfassend eine Vielzahl von Lichtquellen für die Medizin nach einem der Ansprüche 1 bis 6.

9. Lichtquellensystem für die Medizin, umfassend eine erste Lichtquelle, die die Lichtquelle für die Medizin nach einem der Ansprüche 1 bis 6 ist, und eine zweite Weißlichtquelle, die ein von der ersten Lichtquelle verschiedenes Emissionsspektrum aufweist.

10. Lichtquellensystem für die Medizin nach Anspruch 9, wobei die Beleuchtung der ersten Lichtquelle und der zweiten Lichtquelle schaltbar ist.

## Revendications

1. Source de lumière blanche pour application médicale qui est destinée à être utilisée pour l'observation visuelle d'un vaisseau sanguin dans la peau,
la source de lumière ayant un spectre d'émission, dans laquelle la source de lumière blanche comprend une puce à DEL ayant un pic d'émission dans une région de longueurs d'onde de 370 nm à 480 nm ; et une couche de luminophore qui est excitée par une lumière primaire irradiée par la puce à DEL et émet une lumière secondaire dans une région de lumière visible, et dans laquelle,
dans le spectre d'émission, un rapport de P3 à P2 (P3/P2) est de 0,4 ou moins, dans laquelle P3 est l'intensité maximale du spectre d'émission dans une région de longueurs d'onde de 520 nm à 580 nm, et P2 est l'intensité maximale du spectre d'émission dans une région de longueurs d'onde de 600 nm à 780 nm,
dans laquelle un rapport de P1 à P2 (P1/P2) est de 0,5 à 1,2, dans laquelle P1 est l'intensité maximale du spectre d'émission dans une région de longueurs d'onde de 370 nm à 470 nm.

2. Source de lumière blanche pour application médicale selon la revendication 1, dans laquelle le rapport (P3/P2) est de 15 % ou moins.

3. Source de lumière pour application médicale selon la revendication 1 ou la revendication 2, dans laquelle un rapport de S2 à S1 (S2/S1) est de 0,3 ou moins (incluant zéro), dans laquelle S1 est la zone du spectre d'émission dans une région de longueurs d'onde de 370 nm à 780 nm, et S2 est la zone du spectre d'émission dans une région de longueurs d'onde de 471 nm à 599 nm.

4. Source de lumière pour application médicale selon l'une des revendications précédentes, comprenant une pluralité de puces à DEL et ayant une structure dans laquelle la couche de luminophore recouvre une circonférence des puces à DEL.

5. Source de lumière pour application médicale selon l'une des revendications précédentes, comprenant une couche de résine transparente entre la puce à DEL et la couche de luminophore.

6. Source de lumière pour application médicale selon la revendication 5, dans laquelle la couche de luminophore présente une épaisseur de film d'au moins 0,07 mm ou plus, et la couche de résine transparente présente une épaisseur de film de 0,1 à 1,0 mm.

7. Lampe fluorescente utilisant la source de lumière pour application médicale selon la revendication 1 ou la revendication 2.

8. Système de source de lumière pour application médicale, comprenant une pluralité des sources de lumière pour la médecine selon l'une quelconque des revendications 1 à 6.

9. Système de source de lumière pour application médicale, comprenant une première source de lumière qui est la source de lumière pour application médicale selon l'une quelconque des revendications 1 à 6, et une deuxième source de lumière blanche ayant un spectre d'émission différent de celui de la première source de lumière.

10. Système de source de lumière pour application médicale selon la revendication 9, dans lequel l'éclairage de la première source de lumière et de la deuxième source de lumière peuvent être échangés.
